(19) **Europäisches Patentamt**

**European Patent Office**

**Office européen des brevets**

(11) **EP 0 467 853 B1**

(12) **EUROPÄISCHE PATENTSCHRIFT**

(45) Veröffentlichungstag und Bekanntmachung des Hinweises auf die Patenterteilung:
**10.01.1996 Patentblatt 1996/02**

(51) Int. Cl.$^6$: **A61B 5/026**, A61B 5/0285, A61B 8/06

(21) Anmeldenummer: **91810569.3**

(22) Anmeldetag: **15.07.1991**

(54) **Einrichtung und Verfahren zur Blutdruckmessung**

Device and method for the measurement of blood pressure

Dispositif et procédé destinés à la mesure de la pression sanguine

(84) Benannte Vertragsstaaten:
**AT CH DE FR GB LI**

(30) Priorität: **18.07.1990 CH 2390/90**

(43) Veröffentlichungstag der Anmeldung:
**22.01.1992 Patentblatt 1992/04**

(73) Patentinhaber: **AVL Medical Instruments AG**
**CH-8207 Schaffhausen (CH)**

(72) Erfinder: **Hatschek, Rudolf A., Dr.**
**CH-1700 Fribourg (CH)**

(74) Vertreter: **Krause, Walter, Dr. Dipl.-Ing. et al**
**A-1014 Wien (AT)**

(56) Entgegenhaltungen:
**EP-A- 0 027 215        DE-A- 1 905 620**
**FR-A- 2 481 917        FR-A- 2 523 432**
**US-A- 4 370 985**

## Beschreibung

Die Erfindung betrifft eine Einrichtung und ein Verfahren zur Blutdruckmessung mit Sensormitteln zum Anbringen an einem lebenden Körper.

Die Einrichtung und das Verfahren dienen zur Blutdruckmessung, und zwar zur nicht-invasiven Blutdruckmessung. Der Begriff "nicht-invasiv" besagt hierbei, daß die Messung ohne in ein Blutgefäß eingeführtes Instrument und also mit Sensormitteln durchgeführt wird, die sich vollständig außerhalb des lebenden, menschlichen oder eventuell tierischen Körpers befinden, an dem die Blutdruckmessung durchgeführt wird.

Der Blutdruck wird derzeit meistens mit Verfahren gemessen, die auf der Methode von Riva-Rocci basieren. Bekannte, für derartige Blutdruckmessungen vorgesehene Einrichtungen weisen eine deformierbare Manschette auf. Diese begrenzt einen Hohlraum, der mit einer üblicherweise durch eine Pumpe zum Pumpen von Luft gebildeten Druckgasquelle, einem Auslaß und einer Druckmeßvorrichtung verbunden ist. Ferner sind Mittel vorhanden, um beim Ändern des in der Manschette herrschenden Drucks - nämlich beim Entlüften der Manschette - zwei Werte dieses Drucks dem systolischen und dem diastolischen Druck zuordnen zu können. Die Zuordnung zum systolischen und zum diastolischen Druck kann dabei entweder aufgrund der vom Blut beim Durchströmen einer Arterie erzeugten Korotkoff-Töne oder nach der oszillometrischen Verfahrensvariante erfolgen. Bei bekannten, neueren Blutdruckmeßeinrichtungen weisen die Druckmeßvorrichtungen einen mit dem Hohlraum der Manschette verbundenen Meßwandler zur Umwandlung des Druckes in eine elektrische Größe, elektronische Schaltungsmittel und ein Anzeigeorgan zur analogen oder digitalen Anzeige des systolischen und diastolischen Blutdrucks auf. Einrichtungen für die Ermittlung des systolischen und diastolischen Blutdrucks aufgrund der Korotkoff-Töne weisen zudem entweder ein Stethoskop oder ein Mikrofon auf. Es sei hierbei zum Beispiel auf die deutsche Offenlegungsschrift 30 14 199 und die dieser entsprechenden US Patentschrift 4 459 991 verwiesen. Das pulsierende Fliessen des Blutes kann Schwingungen des in der Manschette vorhandenen, normalerweise aus Luft bestehenden Gases erregen. Bei den für die oszillometrische Messung vorgesehene Einrichtungen sind der Druck-Messwandler sowie die elektronischen Schaltungsmittel ausgebildet, um die mit den besagten Schwingungen verbundenen Schwankungen des in der Manschette herrschenden Drucks zu erfassen.

Beim Messen nach der Methode von Riva-Rocci wird die Manschette an einem Körperteil - zum Beispiel an einem Oberarm oder Finger befestigt und aufgepumpt, bis der Druck der in ihrem Hohlraum vorhandenen Luft ausreicht, um die Arterie im umschlossenen Glied abzuschnüren. Danach wird die Manschette langsam entlüftet. Bei der Variante mit Erfassung der Korotkoff-Töne mittels eines Stethoskops oder Mikrofons werden zwei Werte des während des Entlüftungsvorgangs im Hohlraum der Manschette herrschenden Drucks aufgrund der Geräusche als systolischer bzw. diastolischer Blutdruck identifiziert und erfasst. Dabei wird der beim ersten Auftreten von Korotkoff-Tönen in der Manschette herrschende Druck dem systolischen Blutdruck zugeordnet. Der diastolische Druck wird daran erkannt, dass die eigentlichen Korotkoff-Töne verschwinden, wobei die von der Blutströmung erzeugten Geräusche leiser und dumpfer werden oder ganz verschwinden. Bei der oszillometrischen Verfahrensvariante werden die den systolischen und den diastolischen Blutdrücken entsprechenden Drücke der in der Manschette enthaltenen Luft dadurch ermittelt, dass die durch das pulsierende Fliessen des Blutes verursachten Schwankungen des Manschettendrucks erstmals erscheinen bzw. wieder verschwinden.

Bei schwer kranken oder schwer verunfallten und/oder frisch operierten Patienten sowie in anderen Fällen kann es notwendig oder zumindest wünschenswert sein, den Blutdruck des betreffenden Patienten während einer gewissen Zeitdauer - zum Beispiel während mehrerer Stunden oder Tage - dauernd und möglichst kontinuierlich zu überwachen. Aus der Praxis sind für diesen Zweck vorgesehene, nach der Methode von Riva-Rocci arbeitende Einrichtungen bekannt, bei denen die Manschette beim Betrieb automatisch zyklisch aufgepumpt und entlüftet werden kann, wobei während der Entlüftung jeweils der systolische und diastolische Blutdruck gemessen wird. Ein periodisches Aufpumpen sowie anschliessendes Entlüften der Manschette und die dabei jeweils stattfindende Unterbindung der Blutzirkulation im mit der Manschette versehenen Glied ist jedoch für den untersuchten Patienten unangenehm und eventuell sogar gesundheitsschädlich. Da ein Aufpump/Entlüftungszyklus meistens mindestens etwa eine Minute benötigt und da zudem zwischen aufeinanderfolgenden Messungen kleine Pausen eingeschaltet werden sollten, um die Störung des untersuchten Patienten möglichst gering zu halten, ermöglicht die Methode nach Riva-Rocci ferner gar keine wirklich kontinuierliche Blutdruckmessung.

Die Publikation "Possible determinants of pulse-wave velocity in vivo", Masahiko Okada, IEEE Transactions on Biomedical Engineering, Vol. 35, Nr. 5, May 1988, Seiten 357 bis 361, offenbart ein photoplethysmographisches Verfahren für die Messung der noch näher erörterten Pulswellengeschwindigkeit. Die Messung erfolgt unter Verwendung von Licht mit einer Wellenlänge von 300 nm bis 500 nm an den Finger- oder Zehenspitzen. In dieser Publikation wird die Korrelation der Pulswellengeschwindigkeit mit verschiedenen anderen Parametern und Variablen beschrieben, unter denen sich auch der Blutdruck befindet. Gemäss dieser Publikation wurde eine gewisse Korrelation zwischen der Pulswellengeschwindigkeit und dem systolischen sowie diastolischen Blutdruck festgestellt. Eine solche, relativ geringe Korrelation ermöglicht aber keine Bestimmung des Blutdruckes. Da die Pulswellengeschwindigkeit nicht periodisch ändert, wäre es insbesondere auch nicht möglich, aus der Pulswellengeschwindigkeit den systolischen und den diastolischen Blutdruck zu ermitteln. Im übrigen sind die Wände der großen Arterien und die diese meistens gegen außen abdeckenden Gewebeteile für eine Wellenlänge von 300 nm bis 500 nm aufweisendes Licht praktisch undurchlässig. Das aus der Publikation von M. Okada bekannte Verfahren ist daher nur für Messungen an dünnwandigen, oberflächennahen und dementsprechend kleinen Blutgefäßen und nicht für Messungen an großen, entsprechend dickwandigen und eventuell relativ weit von der Oberfläche des untersuchten Körperteils entfernten Blutgefäßen geeignet.

In der FR-A-2 523 432 wird eine kompakte, tragbare Vorrichtung zur Messung des arteriellen Blutdruckes beschrieben, welche eine Einrichtung zur Messung der Strömungsgeschwindigkeit des Blutes mit Hilfe des Dopplereffektes aufweist. Die Einrichtung kann im Bereich einer Arterie befestigt werden. Weiters sind Mittel vorgesehen, die einen Zusammenhang zwischen den gemessenen Werten der Strömungsgeschwindigkeit und dem arteriellen Blutdruck herstellen. Mit der Strömungsgeschwindigkeit allein kann jedoch der Blutdruck nur sehr ungenau bestimmt werden.

Der Erfindung liegt daher die Aufgabe zugrunde, eine Einrichtung und ein Verfahren zur nicht-invasiven Blutdruckmessung zu schaffen, wobei die Einrichtung und das Verfahren Nachteile der bekannten Einrichtungen bzw. Verfahren vermeiden und insbesondere ermöglichen soll, den Blutdruck eines Menschen oder eventuell Tieres im wesentlichen kontinuierlich zu überwachen, ohne daß abwechselnd eine Manschette aufgepumpt sowie entlüftet werden muß, wobei trotzdem eine gute Meßgenauigkeit erreicht werden soll.

Im folgenden werden zunächst einige allgemeine, den Blutkreislauf betreffende Eigenschaften erörtert. Der Blutkreislauf weist arterielle Blutgefäße - d.h. Arterien -, venöse Blutgefäße und die beiden Gefäßarten miteinander verbindende Kapillargefäße auf. Die kleinsten, unmittelbar mit den Kapillargefäßen verbundenen, arterielen Blutgefäße oder Arterien werden als Arteriolen bezeichnet. Die arteriellen Blutgefäße haben elastisch deformierbare Wände und sind mindestens zum Teil mit Muskelfasern versehen und/oder von solchen umschlossen. Diese Muskelfasern können die Arterien und insbesondere die Arteriolen unterschiedlich stark zusammendrücken und dadurch die Elastizität, den Strömungswiderstand sowie die Blutverteilung auf die verschiedenen Blutgefässe beeinflussen. Das Herz pumpt das Blut pulsierend - d.h. stossweise - durch die Blutgefässe. Das Blut strömt mit einer Strömungsgeschwindigkeit v durch die Blutgefässe, die sowohl vom Ort als auch von der Zeit abhängig ist. Wenn man zur Vereinfachung zunächst annimmt, dass die Blutgefässe starre Wände haben, breiten sich Druckänderungen im Blut mit der Schallgeschwindigkeit $c_s$ aus, deren zweite Potenz gegeben ist durch die Formel

$$c_s^2 = K / rho \qquad (1).$$

Hierbei ist rho die Dichte des Blutes und K der Kompressionsmodul, der auch als Volumenelastizitätsmodul bezeichnet wird und gleich dem Reziprokwert der üblicherweise mit kappa bezeichneten Kompressibilität ist.

In Wirklichkeit haben die arteriellen Blutgefässe jedoch nicht starre Wände, sondern - wie bereits erwähnt - elastisch deformierbare Wände. Die arteriellen Blutgefässe werden bei jedem durch einen Herzschlag verursachten Blutstoss und der damit verbundenen impulsartigen Druckerhöhung erweitert. Diese Erweiterungen breiten sich entlang den arteriellen Blutgefässen aus. Die Geschwindigkeit, mit der sich eine durch einen Herzschlag oder Blutstoss verursachte Druckänderung entlang einem arteriellen Blutgefäss unter der Einwirkung von dessen Wandelastizität ausbreitet, bildet die schon erwähnte Pulswellengeschwindigkeit $c_{pw}$. Gemäss dem Buch "Führer durch die Strömungslehre", Ludwig Prandtl, Verlag Friedr. Vieweg & Sohn, Braunschweig, 1965, ist die zweite Potenz der Ausbreitungsgeschwindigkeit von Druckänderungen in Rohren mit elastisch dehnbaren Wänden bei Vernachlässigung von Biegeschwingungen und damit mindestens näherungsweise auch die zweite Potenz der Pulswellengeschwindigkeit gegeben durch die Formel

$$c_{pw}^2 = c_s^2 \, E \, s / (K \, d + E \, s) \qquad (2).$$

Dabei ist E der Elastizitätsmodul der Blutgefässwand, s die Dicke der Blutgefässwand und d der Innendurchmesser des Blutgefässes.

Gemäss der schon zitierten Publikation von M. Okada ist die Pulswellengeschwindigkeit im Quadrat gegeben durch die Formel

$$c_{pw}^2 = E \, s / d \, rho \qquad (3).$$

Durch Einsetzen von $c_s$ in die Formel (2) kann man zeigen, dass die Formel (3) aus der Formel (2) hervorgeht, wenn man in der letzteren zur Vereinfachung im Klammerausdruck den zweiten Summanden weglässt.

Die Strömungsgeschwindigkeit des Blutes ist - wie schon erwähnt - sowohl orts- als auch zeitabhängig. Ihr Maximalwert beträgt in einem arteriellen Blutgefäss und insbesondere in einer grossen Arterie eines erwachsenen Menschen höchstens etwa 0,5 m/s und normalerweise etwas weniger. Die Pulswellengeschwindigkeit ist gemäss den Formen (2) und (3) vom Verhältnis zwischen der Wanddicke und dem Durchmesser der Arterien abhängig. Da sich dieses vom Herzen zu den Kapillargefässen hin vergrössert und da die Pulswellengeschwindigkeit zudem vom Elastizitätsmodul und von der Spannung der zum betreffenden Blutgefäss gehörenden Muskelfasern abhängig ist, ändert sich die Pulswellengeschwindigkeit entlang den arteriellen Blutgefässen und ist auch vom Zustand der untersuchten Menschen oder Tiere abhängig. In den Arterien beträgt die Pulswellengeschwindigkeit typischerweise etwa 4 m/s bis 5 m/s. Die Schallgeschwindigkeit in Wasser, das ja den Hauptbestandteil des Blutes bildet, liegt in der Grösse von 1500 m/s. Die Pulswellengeschwindigkeit $c_{pw}$ ist also wesentlich, nämlich mindestens oder ungefähr 10 Mal grösser als die Strömungsgeschwindigkeit geschwindigkeit v und die Schallgeschwindigkeit $c_s$ ist wiederum sehr viel größer als die Pulswellengeschwindigkeit.

Der sich in einem bestimmten Blutgefäß ergebende Blutdruck hängt von der Pumpleistung des Herzens, vom Strömungswiderstand des Blutgefäßes, von der momentanen Durchflußmenge, von der Elastizität der Blutgefäßwand und von der Viskosität des Blutes ab.

Die gestellte Aufgabe wird durch eine Einrichtung und ein Verfahren mit den Merkmalen des Anspruches 1 bzw. 13 gelöst. Vorteilhafte Ausgestaltungen der Einrichtung und des Verfahrens gehen aus den abhängigen Ansprüchen 2 bis 12 sowie 14 hervor.

Es wurde nämlich gefunden, daß sich der Blutdruck relativ genau dadurch bestimmen läßt, daß zwei verschiedene Größen ermittelt werden, wobei als eine erste Größe bei mindestens einem ein arterielles Blutgefäß enthaltenden Meßbereich kontinuierlich eine zeitlich periodisch im Takt des Pulses ändernde Variable und/oder deren pulsbedingte Änderung und als andere, zweite Größe eine Größe ermittelt wird, die ein Maß für die Pulswellengeschwindigkeit und/oder deren Änderung gibt. Unter Verwendung von mindestens einem nach der weiter vorne beschriebenen Methode von Riva-Rocci ermittelten Eichwert kann dann durch Verknüpfen der beiden Größen mindestens eine Größe gebildet werden, die ein Maß für einen charakteristischen Wert des Blutdrucks und/oder dessen Änderung gibt, wobei zum Beispiel mindestens der systolische und zum Beispiel auch noch der diastolische und/oder mittlere Blutdruck gemessen und angezeigt werden kann.

Die Einrichtung besitzt Sensormittel, die zum Beispiel mindestens einen lösbar an einen Körperteil befestigten Sensor aufweisen, wobei etwa zwei gleichartige oder zwei verschiedenartige Sensoren vorhanden sein können. Zumindest der bzw. jeder zur Messung der ersten, periodisch ändernden Größe dienende Sensor wird vorzugsweise an einem Arm oder eventuell einem Bein befestigt. Die Einrichtung besitzt ferner vorzugsweise eine durch ein Gerät oder mehrere solche gebildete Anzeige- und Überwachungsvorrichtung, die mindestens einen Teil der elektronischen Schaltungsmittel der Einrichtung enthält.

Die genannte erste, zeitlich synchron mit dem Puls ändernde Grösse ändert auch synchron zum Blutdruck - d.h. im gleichen Takt wie dieser - und ist mit dem Blutdruck durch eine physikalische Verknüpfung korreliert, soll aber selbstverständlich verschieden vom Blutdruck und nicht bereits direkt durch den Blutdruck selbst bzw. dessen Änderung gebildet sein. Die Sensormittel und elektronischen Schaltungsmittel können ausgebildet sein, um als erste Grösse eine Grösse zu ermitteln und durch ein elektrisches Signal darzustellen, die ein Mass für den momentanen Wert und/oder die synchron mit dem Puls erfolgende Änderung der Strömungsgeschwindigkeit und/oder der Durchflussmenge und/oder des Volumens des Blutes in einem Messbereich und/oder einer Querschnittsabmessung und/oder der Durchlassquerschnittsfläche mindestens eines arteriellen Blutgefässes gibt. Da die Blutgefässe und insbesondere die Arterien im Querschnitt normalerweise mindestens annähernd kreisförmig sind, kann die genannte, allenfalls ermittelte Querschnittsabmessung mindestens näherungsweise durch den Innen- oder Aussendurchmesser oder einen mittleren Durchmesser des Blutgefässes gebildet sein. Es sei hierzu noch angemerkt, dass die für die erste Grösse angegebenen Variablen eng miteinander verknüpft sind. Wenn man die Durchflussmenge in Volumeneinheiten pro Zeiteinheit misst, so ist die Durchflussmenge nämlich gleich dem Produkt der über die Querschnittsfläche gemittelten, mittleren Strömungsgeschwindigkeit mal der Durchlassquerschnittsfläche des Blutgefässes.

Die Einrichtung kann zum Beispiel ausgebildet sein, um als erste Grösse die Strömungsgeschwindigkeit und/oder Durchflussmenge zu erfassen, indem Lichtwellen - nämlich monochromatische, kohärente Lichtwellen - oder Ultraschallwellen in einen zu untersuchenden Körperteil eingestrahlt und vom Blut oder - genauer gesagt - von den Blutkörperchen gestreute Lichtwellen bzw. Ultraschallwellen aufgefangen werden. Die Einstrahlung der Licht- bzw. Ultraschallwellen kann dabei impulsweise erfolgen. Bei diesen auf der Streuung von Licht oder Ultraschall beruhenden Verfahren können je nach Wunsch rechtwinklig und/oder parallel zur Einstrahlungsrichtung des Lichts bzw. Ultraschalls gerichtete Komponenten der Strömungsgeschwindigkeit oder des Durchflusses erfasst werden. Es sind verschiedene Arten von solchen auf der Streuung von Licht oder Ultraschall basierende Messmethoden bekannt. Bei Verwendung von Licht können die Messungen zum Beispiel mit Hilfe der Photonen-Korrelation, der Licht-Schwebungs-Spektroskopie, (englisch: "light beating spectroscopy"), der Tupfen-Interferometrie (englisch: "speckle interferometry") oder des Dopplereffekts erfolgen. Es sei hierzu beispielsweise auf die Publikation "Laser doppler anemometry, a comparative study of the measurement of motion by light scattering", E.R. Pike, in "The engineering uses of coherent optics", "Proceedings and edited discussion of a conference held at the University of Strathclyde, Glasgow" 8. - 11. April 1975, Seiten 431 bis 457, Cambridge University Press, verwiesen.

Bei der Verwendung von Ultraschall können die Messungen auf ähnliche Arten, nämlich zum Beispiel mit Hilfe der Zeit-Domänen-Korrelation (englisch: "time domain correlation"), des Interferenz-Tupfen-Musters (englisch: "interference speckle pattern") und/oder der Fourier-Transformation oder des Dopplereffekts erfolgen. Einige solche Messmethoden sind zum Beispiel in folgenden Aufsätzen in den "Proceedings zum IEEE Ultrasonics Symposium" 1990, Band 3 beschrieben: "An improved blood velocity estimator optimized for real-time ultrasound flow applications", M.R. Sturgill, R.H. Love, B.K. Herres, Seiten 1467 bis 1471; "Preliminary study into high velocity transverse blood flow measurement", H.F. Routh, T.L. Pusateri, D.D. Waters, Seiten 1523 bis 1526 und "Determination of 2-D velocity vectors using color Doppler ultrasound", T. Tamura, R.S.C. Cobbold, K.W. Johnston, Seiten 1537 bis 1540. Ferner sei noch die Publikation "A novel method for angle independent ultrasonic imaging of blood flow and tissue motion", L.N. Bohs, G.E. Trahey, IEEE Transactions on Biomedical Engineering, Band 38, No. 3, 1991, Seiten 280 bis 286, hingewiesen.

Zu den verschiedenen auf der Streuung von Lichtwellen oder Ultraschallwellen beruhenden Methoden sei noch angemerkt, dass diese von den verschiedenen Autoren und Geräteherstellern zum Teil etwas uneinheitlich bezeichnet werden. Beispielsweise werden den Dopplereffekt-Verfahren zum Teil auch noch Verfahren zugeordnet, bei denen eine zur Strahlungsrichtung rechtwinklige Strömungsgeschwindigkeit gemessen wird, obschon unter dem Dopplereffekt strenggenommen und im klassischen Sinn die Erzeugung einer Frequenzverschiebung durch eine zur Wellenausbreitungsrichtung parallele Geschwindigkeitskomponente einer Wellenstrahlungsquelle verstanden wird.

Falls eine Ermittlung der Strömungsgeschwindigkeit und/oder Durchflussmenge mittels Licht vorgesehen ist, kann die Einrichtung einen oder mehrere Lichtstrahler und einen oder mehrere Lichtempfänger aufweisen. Der oder jeder Sensor kann dann zum Beispiel mindestens einen als Lichtstrahler dienenden, optoelektronischen Wandler, zum Beispiel eine Laser-Leuchtdiode, und mindestens einen als Lichtempfänger dienenden, optoelektronischen Wandler, zum Beispiel eine Photodiode oder einen Phototransistor, aufweisen. Diese Wandler können dann über ein flexibles, elektrisches Kabel mit dem oder einem Gerät der Anzeige- und Überwachungsvorrichtung verbunden sein. Es besteht jedoch auch die Möglichkeit, mindestens einen oder jeden optoelektronischen Wandler im genannten Gerät anzuordnen und über einen flexiblen Lichtleiter mit einem Sensor zu verbinden. In diesem Fall enthalten dann die Sensoren als Lichtstrahler und/oder Lichtempfänger bzw. von diesen nur noch das eine Ende des betreffenden Lichtleiters und beispielsweise noch ein optisches Übertragungselement.

Bei einer vorteilhaften Ausgestaltung einer Einrichtung zur Messung der Strömungsgeschwindigkeit oder Durchflussmenge mit Hilfe der Lichtstreuung wird mit mindestens einem Lichtstrahler Licht erzeugt, dessen Wellenlänge im nahen Infrarotbereich liegt und mindestens 700 nm, höchstens 1200 nm und zum Beispiel 800 nm bis 1000 nm beträgt. Derartiges Licht kann relativ dicke Gewebeschichten und vor allem auch die Wände grosser Arterien durchdringen, so dass die Strömungsgeschwindigkeit und/oder Durchflussmenge nicht nur in kleinen, sondern auch in grossen Arterien gemessen werden kann.

Bei der Ermittlung der Strömungsgeschwindigkeit und/oder Durchflussmenge unter Verwendung von Ultraschall kann der oder jeder Sensor mindestens einen Wandler aufweisen, der ein piezoelektrisches Element besitzt und sowohl einen Ultraschallstrahler zum Einstrahlen von Ultraschallimpulsen in einen Körperteil als auch einen Ultraschallempfänger zum Aufnehmen von zurückgestreutem Ultraschall bildet.

Eine andere Möglichkeit besteht darin, die Einrichtung auszubilden, um aufgrund einer Messung der elektrischen Impedanz oder Admittanz eine erste Grösse zu ermitteln, die ein Mass für das zeitlich ändernde Volumen des in einem Messbereich eines Körperteils vorhandenen Blutes und/oder des Durchmessers und/oder der Durchlassquerschnittsfläche von mindestens einer im Messbereich vorhandenen Arterie und/oder von der Blut-Durchflussmenge gibt. Die Einrichtung kann Sensormittel mit Elektroden aufweisen, die entlang einer im betreffenden Körperteil vorhandenen, grossen Arterie in Abstand voneinander derart lösbar am betreffenden Körperteil befestigt werden, dass sie diesen mindestens zum grössten Teil und beispielsweise vollständig umschliessen. Die Einrichtung besitzt vorzugsweise einen Hochfrequenz-Generator, um eine Wechselspannung zu erzeugen und mindestens zwei Elektroden zuzuführen. Die Frequenz der Wechselspannung kann etwa im Bereich von 30 kHz bis 3 MHz liegen und beträgt nämlich vorzugsweise mindestens 70 kHz, höchstens 150 kHz und beispielsweise 80 kHz bis 100 kHz. Für weitere allgemeine Information zu dieser Variante der Messmethode sei auf die Publikationen "The measurement of peripheral blood flow by the electrical inpedance technique", S.N. Mohapatra, H.M. Arenson, Journal of Medical Engineering and Technology, Band 3, No. 3, 1979, Seite 132 bis 137 und "Digital enhancement of the peripheral admittance plethysmogram", L.A. Marks, IEEE Transactions on Biomedical Engineering, Band BME-34, No. 3, 1987, Seiten 192 bis 197, verwiesen.

Die Strömungsgeschwindigkeit des Blutes kann auch induktiv mit mindestens eine Spule aufweisenden Sensormitteln gemessen werden. Die bzw. jede Spule kann derart lösbar an einem Arm oder andern Körperteil befestigt werden, dass die Spulenachse ungefähr rechtwinklig zu einer grossen Arterie verläuft und diese vorzugsweise mindestens annähernd kreuzt.

Ferner kann als erste Grösse eine ein Mass für den Durchmesser einer grossen Arterie gebende Grösse ermittelt werden, indem die Reflexion von Ultraschall an der Arterienwand verwertet wird. Aus dem Durchmesser kann dann selbstverständlich auch die Durchlassquerschnittsfläche der Arterie bestimmt werden.

Zur Bestimmung der Pulswellengeschwindigkeit und/oder der zeitlichen Änderung von dieser bestehen ebenfalls verschiedene Möglichkeiten. Die Sensormittel und elektronischen Schaltungsmittel können zum Beispiel ausgebildet sein, um die erste, synchron zum Puls zeitlich ändernde Grösse - d.h. Variable - bei zwei entlang dem Strömungsweg des arteriellen Blutes, insbesondere entlang einer grossen Arterie, voneinander im Abstand stehenden Mess-Stellen oder Messbereiche zu ermitteln und durch elektrische Signale darzustellen. Des weitern kann die Zeitdifferenz ermittelt werden, um welche die zeitlichen Verläufe der bei den zwei Messbereichen ermittelten, ersten Grösse - beispielsweise die Maxima der ersten Grösse - gegeneinander verschoben sind. Bei festgelegtem Abstand der beiden Messbereiche ist die Pulswellengeschwindigkeit dann umgekehrt proportional zur besagten Zeitdifferenz. Die zweite Grösse kann ebenfalls in Form eines elektrischen Signals dargestellt werden und also beispielsweise durch die genannte Zeitdifferenz und/oder durch deren Reziprokwert und/oder direkt durch die Pulswellengeschwindigkeit gebildet sein.

Die von einem Herzen erzeugten Pulswellen breiten sich von diesem weg entlang von arteriellen Blutgefässen zu den Kapillargefässen aus und werden von diesen mit mehr oder weniger stark reduzierter Amplitude reflektiert. Wie nun erläutert wird, ermöglicht diese Reflexion der Pulswellen, aus dem zeitlichen Verlauf der bei einem einzigen Messbereich gemessenen, ersten Grösse eine zweite Grösse abzuleiten, die ein Mass für die Pulswellengeschwindigkeit gibt. Eine entlang einer Arterie vom Herzen weglaufende Pulswelle wird im folgenden als primäre Pulswelle bezeichnet. Da eine grosse Arterie - wie etwa diejenige eines Arms oder Beins - normalerweise über mehrere entlang von ihr verteilte Verzweigungen mit Kapillargefässen verbunden ist, können von verschiedenen Kapillargefässen reflektierte Pulswellen zur genannten, grossen Arterie zurückgelangen und sich in dieser zu einer grössern, reflektierten Pulswelle überlagern, die ihrerseits der primären Pulswelle überlagert wird und also mit dieser interferiert. Der sich bei einer bestimmten Mess-Stelle der grossen Arterie ergebende Gangunterschied zwischen der primären Pulswelle und der reflektierten Pulswelle ist von der Pulswellengeschwindigkeit und von der Entfernung der die Reflexion bewirkenden Kapillargefässe vom Messbereich abhängig. Wenn sich der Messbereich an einem Arm befindet, erfolgt die Reflexion vor allem in der viele Kapillargefässe enthaltenden Hand, so dass der besagte Gangunterschied massgeblich durch die Entfernung der Hand vom Messbereich bestimmt wird. Bei einer Messung an einem Bein, ist analog die Entfernung des Fusses vom Messbereich massgebend für den Gangunterschied. Die den zeitlichen Verlauf des Blutdruckes wiedergebende Kurve hat in jeder Periode ein Haupt-Maximum, dessen Wert gleich dem systolischen Blutdruck ist, und ein durch die Interferenz zwischen einer primären und einer reflektierten Pulswelle verursachtes mehr oder weniger ausgeprägtes, kleineres Neben-Maximum, das in der Fachsprache als dikrotischer Knoten (englisch: "dicrotic notch") bezeichnet wird. Im übrigen befindet sich der dikrotische Knoten jeweils im abfallenden Teil der Blutdruckkurve, d.h. zwischen einem systolischen Blutdruckwert und dem nachfolgenden diastolischen Blutdruckwert. Die Strömungsgeschwindigkeit und die andern angegebenen Variablen, welche die erste zu messende Grösse bilden können, werden bei wachsendem Blutdruck grösser und bei abnehmendem Blutdruck kleiner. Dementsprechend haben die zeitlichen Verläufe der als erste Grösse genannten Variablen bei jedem Herzschlag ein dem systolischen Druck zugeordnetes Haupt-Extremum, nämlich Haupt-Maximum, und ein dem dikrotischen Knoten zugeordnetes Neben-Extremum, nämlich Neben-Maximum und dazwischen ein relatives Zwischen-Minimum. Die zeitliche oder phasenmässige Verschiebung zwischen einem Haupt-Extremum und dem diesem zeitlich benachbarten, nachfolgenden Neben-Extremum ist vom Gangunterschied zwischen der primären Pulswelle sowie der reflektierten Pulswelle und damit auch von der Pulswellengeschwindigkeit abhängig. Die Sensormittel und Schaltungsmittel können daher ausgebildet sein, um bei einem Messbereich aus dem zeitlichen Verlauf der für die erste Grösse ermittelten Variablen als zweite Grösse die Zeitdifferenz und/oder Phasenwinkeldifferenz zwischen dem Haupt-Extremum sowie einem benachbarten, nachfolgenden Extremum, nämlich dem Neben-Maximum oder eventuell dem Zwischen-Minimum, zu bilden und in Form eines elektrischen Signals darzustellen.

Eine weitere Möglichkeit zur Ermittlung der Pulswellengeschwindigkeit besteht darin, die Sensormittel und die elektronischen Schaltungsmittel auszubilden, um bei einem Messbereich eine der als erste Grösse genannten Variablen und zusätzlich analog wie bei der Elektrokardiographie mindestens einen Strom des Herzmuskels zu erfassen und die sogenannte R-Zacke des Kardiogramms zu ermitteln, bei welcher die Systole - d.h. die Kontraktion des Herzmuskels - und der Blutausstoss aus dem Herzen erfolgt. Wie schon beschrieben, hat die Strömungsgeschwindigkeit oder sonstige, die erste Grösse bildende Variable während Pulsperiode ein im gleichen Zeitpunkt wie der systolische Blutdruck auftretendes Maximum. Die Einrichtung kann daher ferner ausgebildet sein, um die Zeitdifferenz zwischen bestimmten, vorgegebenen, in jeder Pulsperiode einmal vorhandenen Stellen der Kurven zu ermitteln, welche die zeitlichen Verläufe des Herzmuskelstroms und der als erstgenannten Grösse gemessenen Variablen darstellen. Dabei kann zum Beispiel die Zeitdifferenz zwischen der R-Zacke der Herzmuskelstrom-Kurve und dem Zeitpunkt ermittelt werden, in dem die erste Grösse während einer Pulsperiode ein Maximum hat. Diese Zeitdifferenz oder ihre Änderung und/oder eine mit dieser Zeitdifferenz und deren Änderung verknüpfte Grösse können dann als zweite Grösse dienen, die ein Mass für die Pulswellengeschwindigkeit bzw. deren Änderung gibt.

Der Erfindungsgegenstand wird anschliessend anhand in der Zeichnung beschriebener Ausführungsbeispiele näher erläutert. In der Zeichnung zeigt

die Figur 1 eine schematische Ansicht einer Einrichtung zur Blutdruckmessung mit am Arm einer Person angeordneten Sensormitteln,

die Figur 2 einen schematischen Längsschnitt durch Abschnitte des Arms sowie der an diesem befestigten, Licht-strahler und Lichtempfänger aufweisenden Sensormittel und ein Blockschema elektronischer Schaltungsmittel der Einrichtung,

die Figur 3 einen schematischen Längsschnitt durch Abschnitte eines Arms sowie der an diesem befestigten, Ultraschall-Wandler aufweisenden Sensormittel,

die Figur 4 einen der Figur 3 entsprechenden Längsschnitt durch Abschnitte eines Arms und andersartige Ultraschall-Wandler aufweisende Sensormittel,

die Figur 5 eine schematische Ansicht einer Variante einer Blutdruckmess-Einrichtung mit am Arm einer Person angeordneten Sensormittel, die zwei Sensoren mit Elektroden zur Messung der elektrischen Impedanz oder Admittanz aufweisen,

die Figur 6 eine Draufsicht auf die zum Anliegen am Arm bestimmte Seite von einem vom Arm entfernten Sensor der Einrichtung gemäss der Figur 5 in grösserem Massstab,

die Figur 7 einen stark vereinfachten Querschnitt durch den in der Figur 5 ersichtlichen Arm und einen der an diesem befestigten Sensoren in noch grösserem Massstab als die Figur 6,

die Figur 8 eine schematische Ansicht einer Variante von an einem Arm angeordneten Sensormitteln mit Elektroden für die Messung der elektrischen Impedanz oder Admittanz,

die Figur 9 ein Diagramm in zu Veranschaulichung der Ermittlung der Pulswellengeschwindigkeit aufgrund der Reflexion der Pulswellen und

die Figur 10 eine schematische Ansicht einer Person und einer Blutdruckmess-Einrichtung, die zur Ermittlung der Pulswellengeschwindigkeit durch Erfassen eines Herzmuskelstroms ausgebildet ist.

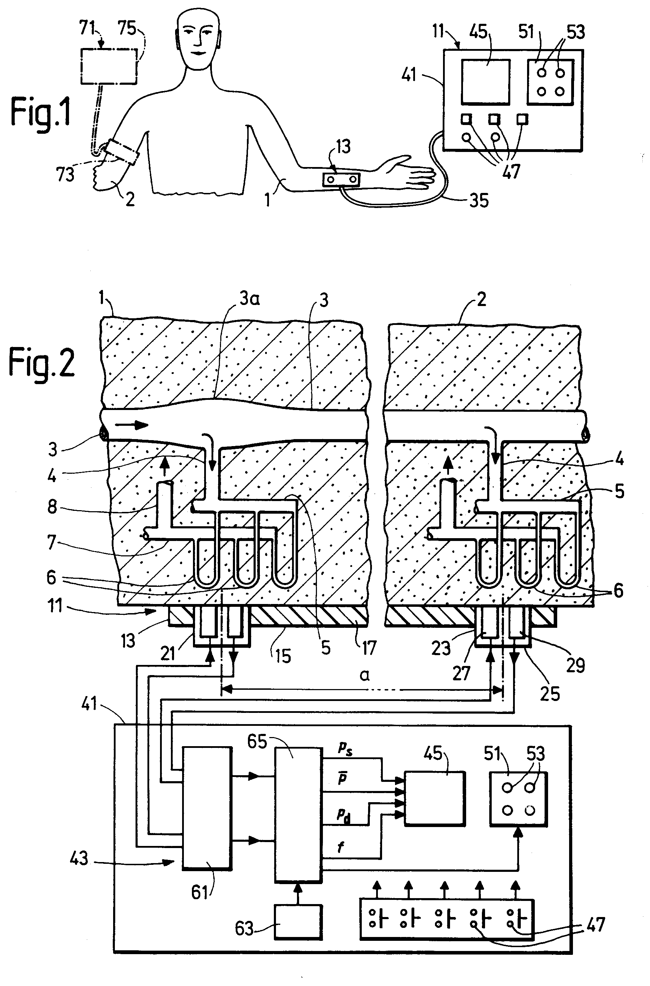

In der Figur 1 ist ein Körperteil 1, nämlich ein Arm 1 eines lebenden, menschlichen Körpers dargestellt. Der zum Teil auch schematisch sowie vereinfacht in der Figur 2 gezeichnete Arm 1 enthält Blutgefässe. Zu diesen gehört eine grosse, entlang dem ganzen Arm verlaufende Arterie 3, von welcher Abschnitte der im Unterarm vorhandenen Arteria radialis gezeichnet sind und die eine durch einen Blutstoss erzeugte, fortschreitende Erweiterung 3a aufweist. Die Arterie 3 ist durch entlang von ihr verteilte und abgezweigte, kleinere Arterien 4 und Arteriolen 5 mit den einen Enden von Kapillargefässen 6 verbunden. Dabei sind insbesondere viele Arteriolen vorhanden, die nahe der Körperoberfläche in und/oder unter der Haut und mindestens annähernd parallel zu dieser verlaufen. Die nicht mit Arteriolen 5 zusammenhängenden Enden der Kapillargefässe 6 sind über Venolen 7 mit grössern Venen 8 verbunden.

Eine in den Figuren 1 und 2 ersichtliche Einrichtung 11 dient zur Blutdruckmessung sowie zur Pulsfrequenzmessung und weist aussen am Arm 1 - nämlich am Unterarm - angeordnete Sensormittel 13 auf. Diese haben einen lösbar am Arm 1 befestigten Halter 15 mit einem länglichen, plättchen- oder streifenförmigen, beispielsweise ein wenig flexiblen, aus elektrisch isolierendem Kunststoff bestehenden Haltekörper 17, dessen Längsrichtung ungefähr parallel zu derjenigen des Arms ist und der auf einer Seite an diesem anliegt. Die lösbare Befestigung des Halters 15 kann zum Beispiel mit nicht gezeichneten, lösbaren Klebeverbindungsmitteln erfolgen, die etwa einen zwischen dem Arm 1 und dem Haltekörper 17 angeordneten, beidseitig klebenden Klebstreifen und/oder mindestens einen auf der Aussenseite des Halters 15 an diesem und auch am Arm angeklebten Klebstreifen aufweisen. Statt Klebeverbindungsmittel kann der Halter 15 auch mit einer den Arm 1 umschliessenden Manschette versehen werden, die einen Klettverschluss oder irgend einen anderen Verschluss aufweist.

Der Halter 15 hält einen ersten Sensor 21 und einen zweiten Sensor 23. Die beiden Sensoren 21, 23 stehen in der Längsrichtung des Arms 1 sowie der Arterie 3 voneinander in Abstand und sind beispielsweise lösbar oder unlösbar am Haltekörper 17 befestigt, etwa festgeklebt. Jeder Sensor 21, 23 besitzt ein beispielsweise im wesentlichen zylindrisches Gehäuse 25, in welchem mindestens ein Lichtstrahler 27 und mindestens ein Lichtempfänger 29 angeordnet sind. Die Lichtstrahler 27 und Lichtempfänger 29 der beiden Sensoren weisen je einen optoelektronischen Wandler, nämlich eine Laser-Leuchtdiode bzw. eine Photodiode auf. Die Laser-Leuchtdioden der Lichtstrahler erzeugen beim Betrieb kohärentes, monochromatisches Licht dessen Wellenlänge 780 bis 820 nm und nämlich beispielsweise ungefähr 810 nm beträgt. Wie schon in der Einleitung dargelegt, kann derartiges, im nahen Infrarotbereich liegendes Licht, die im Arm vorhandenen Gewebe und die Wände aller im Arm 1 vorhandenen Arterien - also insbesondere auch der grossen Arterie 3 - durchdringen. Die Lichtstrahler 27 der beiden Sensoren 21, 23 sind derart angeordnet, dass ihre Strahlungsachsen - d.h. die Mittelachsen der von ihnen in den Arm 1 eingestrahlten Lichtwellenbündel - ungefähr rechtwinklig zur Oberfläche und zur Längsrichtung des Arms 1 und zur Arterie 3 verlaufen. Die Lichtempfänger 29 sind ebenfalls derart angeordnet, dass sie Licht aufnehmen können, das ungefähr rechtwinklig zur Oberfläche des Arms 1 aus diesem austritt. Die Sensoren 21, 23 können beispielsweise durch je einen Lichtstrahler und Lichtempfänger aufweisende Sensoren gebildet sein, wie sie von der Firma Moor Instruments Ltd., Devon, Grossbritannien, für die von dieser Firma unter der Typen-Bezeichnung MBF3D hergestellten Zweikanal-Blutströmungsmonitoren geliefert werden. Die Sensoren 21, 23 können dabei derart angeordnet werden, dass die Strahlungsachsen entweder die grosse Arterie 3 kreuzen oder dies nicht tun.

Der in der Figur 2 mit a bezeichnete Abstand der beiden Sensoren 21, 23 voneinander beträgt vorzugsweise mindestens 3 cm. Insbesondere wenn die Lichtstrahler nicht auf die grosse Arterie 3 ausgerichtet werden, ist es vorteilhaft, wenn der Abstand a relativ gross ist und beispielsweise mindestens oder ungefähr 6 cm beträgt. Wenn die beiden Sensoren gemäss der Figur 1 am Unterarm angeordnet werden, kann der Abstand a beispielsweise bis etwa 10 cm oder eventuell sogar bis etwa 15 cm betragen. Es sei jedoch eingefügt, dass man auch den einen Sensor am Oberarm und den andern Sensor am Unterarm befestigen kann, wobei dann der entlang dem gestreckten Arm gemessene Abstand noch grösser als die vorgängig angegebene Werte sein kann. Die zwei Sensoren 21, 23 haben zum Beispiel einen etwa 7 mm betragenden Durchmesser sowie eine in der Grösse von 5 mm liegende, axiale Abmessung. Die Sensormittel 13 beanspruchen also nur wenig Platz, haben nur ein geringes Gewicht und enthalten im Gegensatz zu den nach der Methode von Riva-Rocci arbeitenden Einrichtungen keine aufzublähende und zu entlüftende Manschette und auch sonst keine beim noch näher beschriebenen Messen zu deformierenden und/oder zu bewegenden Teile, so dass sie den Patienten, an dem sie angebracht sind, nur wenig stören.

Die beiden Sensoren 21, 23 der Sensormittel 13 sind durch ein elektrisches Kabel 35 elektrisch leitend mit einem in Abstand vom untersuchten Menschen etwa auf einem Tisch oder einer Konsole eines Bettgestells angeordneten Anzeige- und Überwachungsvorrichtung 41 verbunden. Diese weist ein Gerät mit einem Gehäuse auf, könnte aber auch durch mehrere Geräte mit separaten Gehäusen gebildet sein. Die Vorrichtung 41 besitzt im und/oder am Gehäuse angeordnete, elektronische Schaltungsmittel 43 mit dem in der Figur 2 gezeichneten Blockschema, mindestens eine Anzeigevorrichtung 45 zur digitalen und/oder analogen Anzeige verschiedener Messgrössen und manuell betätigbare, zum Beispiel drück- und/oder dreh- und/oder kippbare Schalt- und/oder Stellorgane 47. Ferner ist eine Alarmiervorrichtung 51 mit mindestens einem optischen Alarmsignalgeber 53 und nämlich mit mehreren solchen vorhanden, die etwa aus Leuchtdioden und/oder Lämpchen bestehen. Die Alarmiervorrichtung 51 kann ferner noch mindestens einen akustischen Alarmsignalgeber aufweisen und/oder elektrisch mit einem solchen verbunden sein. Falls die Einrichtung in einem Spital benutzt wird, kann übrigens noch vorgesehen werden, dass die Vorrichtungen 41 mehrerer Einrichtungen über elektrische Leiter oder drahtlos Alarmsignale und eventuell Messwerte auf eine zentrale Überwachungs- und Alarmiervorrichtung übertragen können, die optische und/oder akustische Alarmsignalgeber aufweist.

Die elektronischen Schaltungsmittel 43 weisen eine elektrisch leitende mit den optoelektronischen Wandlern der Lichtstrahler 27 und Lichtempfänger 29 der beiden Sensoren 21, 23 verbundene Speise- und Detektorvorrichtung 61 auf. Diese besitzt mindestens eine beispielsweise durch einen Impulsgenerator gebildete Erregerspannungsquelle, um mindestens eine beispielsweise aus einer Impulsfolge bestehende Erregerspannung zu erzeugen und den Lichtstrahlern 27 zuzuführen, so dass diese Licht - beispielsweise Lichtimpulse - in den Arm einstrahlen. Bei der Benutzung der Einrichtung gelangt von den Blutkörperchen des den Arm 1 durchströmenden, arteriellen Blutes reflektiertes oder - genauer gesagt - zurückgestreutes Licht in die Lichtempfänger 29, deren Wandler dieses Licht in elektrische Signale umwandeln. Die Speise- und Detektorvorrichtung 61 besitzt nun des weitern Schaltungsmittel, um aufgrund des rückgestreuten Lichts die Strömungsgeschwindigkeiten des pulsierenden, arteriellen Blutes in den von den beide Sensoren 21, 23 erfassten Messbereichen des Arms 1 zu ermitteln. Die Vorrichtung 61 kann dann elektrische Signale oder Grössen bilden, die in analoger oder digitaler Form ein Mass für die momentanen, Werte der genannten Strömungsgeschwindigkeiten des Blutes geben. Die Speise- und Detektorvorrichtung 61 kann die Strömungsgeschwindigkeiten gleich oder ähnlich ermitteln, wie es in der in der Einleitung zitierten Literatur für quer zur Strömungsrichtung gerichtete Lichtstrahlen beschrieben ist. Die Vorrichtung 61 kann ferner zum Beispiel gleich oder ähnlich ausgebildet sein, wie die Schaltung in den bereits erwähnten, unter der Typen-Bezeichnung MBF3D der Firma Moor Instruments Ltd. erhältlichen Zweikanal-Blutströmungsmonitoren.

Die Anzeige- und Überwachungsvorrichtung besitzt noch eine Eichwerteingabevorrichtung 63 mit elektronischen Schaltungsmitteln, die mit mindestens einem der manuell betätigbaren Betätigungsorgane 47 verbunden sind.

Die Speise- und Detektorvorrichtung 61 ist mit einer Auswertungsvorrichtung 65 verbunden und führt dieser beim Messen die erwähnten, ein Mass für die Strömungsgeschwindigkeiten bei den beiden Sensoren 21, 23 gebenden, elektrischen Signale oder Grössen zu. Die Eichwerteingabevorrichtung 63 ist ebenfalls mit der Auswertungsvorrichtung 65 verbunden, um dieser beim Eichen durch elektrische Signale oder Grössen in analoger oder digitaler Form dargestellte Eichwerte zuzuführen. Die Auswertungsvorrichtung 65 weist vorzugsweise einen Mikroprozessor, mindestens einen Speicher für die Speicherung digital dargestellter Daten und nötigenfalls Analog/Digital- und/oder Digital/Analogwandler auf. Die Auswertungsvorrichtung 65 hat Ausgänge, die mit der Anzeige-vorrichtung 45 und mit der Alarmiervorrichtung 51 verbunden sind. Es sei hier noch angemerkt, dass die im in der Figur 2 ersichtlichen Blockschema gezeichnete Aufteilung der elektronischen Schaltungsmittel 43 auf drei funktionelle Blöcke-d.h. Vorrichtungen 61, 63 und 65 - nur schematisch ist und dass beispielsweise gewisse Teile dieser Blöcke oder Vorrichtungen durch ein- und dieselbe integrierte Schaltung und/oder durch ein und denselben Mikroprozessor gebildet sein können.

Nun sollen noch einige Aspekte der Messung näher erläutert werden. Wie bereits erwähnt, gelangt beim Betrieb der Einrichtung von den Blutkörperchen des arteriellen Bluts reflektiertes bzw. zurückgestreutes Licht in die Lichtempfänger 29 der beiden Sensoren 21, 23. Dabei kann zusätzlich zu dem vom arteriellen Blut reflektierten Licht auch Licht in die Lichtempfänger gelangen, das von venösem Blut oder von Gewebeteilen oder irgendwelchen andern Komponenten des Arms 1 gestreut wurde. Da jedoch von dem in die Lichtempfänger gelangenden Licht nur dasjenige zeitlich im Takt des Pulses variiert, das vom pulsierenden, arteriellen Blut gestreut wurde, kann die Speise- und Detektorvorrichtung 61 den pulsierenden Lichtanteil herausfiltern oder in anderer Weise erkennen und eben die Strömungsgeschwindigkeit des arteriellen Blutes ermitteln.

Wie bereits in der Einleitung erörtert, verursacht das stossweise Pumpen des Blutes periodische und zum Puls synchrone, zeitliche Blutdruckänderungen, die sich mit der Pulswellengeschwindigkeit ausbreiten. Jedes infolge eines Herzschlages entstehende Druckmaximum ist mit einem Maximum der Strömungsgeschwindigkeit verknüpft und verursacht in der Arterie 3 die bereits erwähnte Erweiterung 3a, die mit der Pulswellengeschwindigkeit $c_{pw}$ in der Strömungsrichtung des Blutes entlang der Arterie 3 fortschreitet. Die Strömungsrichtung des Blutes und die Ausbreitungrichtung der Druckänderung sowie der Erweiterung 3a sind in der Figur 2 durch einen Pfeil bezeichnet. Die Erweiterung oder - genauer gesagt - deren Maximum passiert zuerst den ersten Sensor 21 und dann nach einer Laufzeit $T_a$ den im Abstand a vom ersten Sensor stehenden, zweiten Sensor 23. Die sich bei den beiden Sensoren in der Arterie 3 ergebenden Maxima der Strömungsgeschwindigkeiten sind dementsprechend um die Laufzeit $T_a$ zeitlich gegeneinander verschoben. Die beiden Sensoren können - wie erwähnt - derart angeordnet sein, dass die von ihren Lichtstrahlern erzeugten Lichtstrahlenbündel die grosse Arterie 3 kreuzen. Wenn dies zutrifft, kann vom die Arterie 3 durchströmenden Blut reflektiertes Licht in die Lichtempfänger gelangen. In einem solchen Fall kann direkt die Strömungsgeschwindigkeit von die Arterie 3 durchströmendem Blut ermittelt werden. Unter dieser Voraussetzung und unter der Voraussetzung, dass der sich zwischen den beiden Sensoren 21, 23 befindende Abschnitt der Arterie 3 gerade ist sowie parallel zur Verbindungsgeraden zwischen den Zentren der am Arm anliegenden Flächen der beiden Sensoren verläuft, kann die Pulswellengeschwindigkeit errechnet werden gemäss der Formel

$$c_{pw} = a / T_a \qquad (4).$$

In Wirklichkeit ist die Arterie 3 normalerweise nicht genau parallel zur erwähnten, die Sensoren verbindenden Verbindungsgeraden. Die Pulswellengeschwindigkeit ist dann aber immer noch umgekehrt proportional zur Laufzeit $T_a$.

Wie erwähnt, können die Sensoren auch derart angeordnet werden, dass die in den Arm 1 gestrahlten Lichtstrahlenbündel nicht die grosse Arterie 3, sondern nur kleinere arterielle Blutgefässe, insbesondere mehr oder weniger zur Oberfläche des Arms parallele Arteriolen 5, kreuzen. Da diese Arteriolen durch relativ kurze, kleine Arterien 4 mit der grossen Arterie 3 verbunden sind, ist der zeitliche Verlauf der Pulswelle in den Arteriolen 5 nur relativ wenig gegen den zeitlichen Verlauf der Pulswelle in denjenigen Abschnitten der grossen Arterien 3 verschoben, die sich in den Messbereichen befinden, bei denen die Sensoren angeordnet sind. Zumindest wenn der Abstand a so gross gewählt wird, dass die in den Messbereichen der beiden Sensoren vorhandenen Arteriolen bei entlang der Arterie 3 in Abstand voneinander stehenden Abzweigungen mit dieser verbunden sind, ist die Pulswellengeschwindigkeit dann ebenfalls mindestens annähernd durch die Formel (4) bestimmt und jedenfalls noch proportional zum Reziprokwert der Laufzeit $T_a$.

Die Auswertungsschaltung 65 ist ausgebildet, um aus den beiden ihr von der Speise- und Detektorvorrichtung 65 zugeführten Folgen von Signalen die Laufzeit $T_a$ zu ermitteln und ein elektrisches, vorzugsweise digitales Signal zu erzeugen, das ein Mass für die Pulswellengeschwindigkeit gibt. Aus den mit den beiden Sensoren ermittelten Messwerten können also die relativen Werte von zwei Grössen ermittelt und durch elektrische Signale dargestellt werden, von denen die eine ein Mass für die Strömungsgeschwindigkeit bei einem der beiden Sensoren 21, 23 und die andere ein Mass für die Pulswellengeschwindigkeit in der Arterie 3 gibt. Da die Strömungsgeschwindigkeit im Takt des Pulses variiert, können die elektronischen Schaltungsmittel 43 auch die Pulsfrequenz ermitteln. Zudem können die Schaltungsmittel ausgebildet sein, um eventuell noch den Amplitudenwert oder den zeitlichen Mittelwert der pulsierenden Strömungsgeschwindigkeit zu ermitteln und durch ein elektrisches Signal darzustellen. Ferner können die Schaltungsmittel zum Beispiel auch den Mittelwert der bei den beiden Sensoren gemessenen Amplitudenwerte oder zeitlichen Mittelwerte der Strömungsgeschwindigkeiten bilden.

Für das Durchführen einer kontinuierlichen Messung des Blutdrucks wird die Einrichtung 11 mit Hilfe einer zusätzlichen, nur vorübergehend und nur während einer im Vergleich zur ganzen Messdauer kurzen Zeit benutzten, strichpunktiert in der Figur 1 angedeuteten Eich-Messvorrichtung 71 geeicht. Diese weist eine aufblähbare Manschette 73 sowie ein mit dieser verbundenes Messgerät 75 auf und ist zum Messen des Blutdrucks nach der Methode von Riva-Rocci ausgebildet. Zum Eichen wird die Manschette 73 nach dem Befestigen der Sensormittel 13 am Arm 1 vorübergehend an einem Körperteil, und zwar am Oberarm des andern Arms 2 angebracht. Hierzu ist anzumerken, dass die Manschette 73 und die Sensormittel 13 vorzugsweise nicht entlang dem gleichen Blutströmungsweg angeordnet werden sollten, weil das Abschnüren der Blutströmung durch die Manschette und die nach dem Entfernen der Manschette noch während einer gewissen Zeitdauer vorhandenen Nachwirkungen der Abschnürung Messfehler verursachen könnten. Mit Hilfe von einem Schalt- und/oder Stellorgan 47 bringt man dann die Anzeige- und Überwachungsvorrichtung 41 der Einrichtung 11 in einen Eichmodus, d.h. in eine zum Eichen vorgesehene Betriebsart, misst gleichzeitig mit der zusätzlichen Eich-Messvorrichtung 71 nach der Methode von Riva-Rocci in einer der in der Einleitung beschriebenen Arten die Werte des systolischen Blutdrucks $p_s$ sowie des diastolischen Blutdrucks $p_d$ sowie eventuell noch gewisse Zwischenwerte des Blutdrucks. Diese mit der Eich-Messvorrichtung ermittelten Eichwerte werden dann manuell mit mindestens einem der Schalt- und/oder Stellorgane 47 über die Eichwerteingabevorrichtung 63 in die Auswertungsschaltung 65 eingegeben. Es besteht jedoch auch die Möglichkeit, die Anzeige- und Überwachungsvorrichtung 41 und die Eich-Messvorrichtung 71 derart auszubilden, dass die beiden Vorrichtungen 41 und 71 beim Eichen vorübergehend über ein Kabel und Steckverbindungen miteinander verbunden werden können. In diesem Fall kann ferner vorgesehen werden, dass die Eich-Messvorrichtung 71 der Vorrichtung 41 mindestens einen Teil der zum Eichen der Einrichtung 11 erforderlichen Eichwerte automatisch in Form elektrischer Signale zuführt. Wenn die Einrichtung 11 auf die eine oder andere Weise geeicht wurde, kann die Manschette 73 der Eich-Messvorrichtung 71 wieder vom Arm 1 entfernt werden, während die Sensormittel 13 bis zum Ende der vorgesehenen Messdauer am Arm 1 befestigt bleiben.

Wenn sich die Messung über längere Zeit - zum Beispiel über mehrere Tage - erstreckt, kann die Einrichtung 11 mit Hilfe der Eich-Messvorrichtung 71 von Zeit zu Zeit - zum Beispiel ein Mal täglich - neu geeicht werden.

Nun soll erläutert werden, wie aus den gemessenen Werten Strömungsgeschwindigkeit sowie der Pulswellengeschwindigkeit und den Eichwerten der Blutdruck ermittelt werden kann. Hierzu wird zunächst angenommen, dass ein beim Messen erfasstes Blutgefäss aus einem kreiszylindrischen Rohr mit einer starren Wand besteht. Die in Volumeneinheiten pro Zeiteinheit gemessene Durchflussmenge Q durch ein Rohr mit einer starren Wand, dem Innenradius r und der Länge L ist bei laminarer Strömung gemäss dem Gesetz von Hagen-Poiseuille gegeben durch die Formel

$$Q = p_L \, r^4 \, pi \, / \, 8 \, L \, eta \qquad (5) .$$

Dabei bezeichnet $p_L$ die über einem Rohrstück mit der Länge L vorhandene Druckdifferenz und eta den dynamischen Viskositätskoeffizienten.

Wenn die in der Formel (5) auf beiden Seiten des Gleichheitszeichens stehenden Grössen durch die Innen- oder Durchlassquerschnittsfläche F des vorderhand als starrwandiges Rohr betrachteten Blutgefässes dividiert, ergibt sich für die Strömungsgeschwindigkeit

$$v = p_L \, r^2 \, / \, 8 \, L \, eta \qquad (6)$$

Unter der vereinfachenden Annahme, dass die Blutgefässe starre Wände haben, ist der momentane Blutdruck p proportional zur Druckdifferenz $p_L$ und also gemäss den Formeln (5) und (6) proportional zur momentanen Durchflussmenge Q und zur momentanen Strömungsgeschwindigkeit v. Unter der genannten Annahme und unter der Voraussetzung, dass die Viskosität des Blutes während einer Messung konstant bleibt, kann also die Verknüpfung des Blutdrucks p mit der Strömungsgeschwindigkeit v dargestellt werden durch die Formel

$$p = v \, k_v \, / \, r^2 \qquad (7)$$

Dabei bezeichnet $k_v$ eine Konstante. Wenn die Blutgefässwände gemäss der Annahme starr sind, ist auch $r^2$ konstant. Der dann ebenfalls konstante Quotient $k_v/r^2$ könnte dann beim Eichen ermittelt werden. Zum Ermitteln des Blutdrucks müsste man dann lediglich die Strömungsgeschwindigkeit v messen und gemäss der Formel (7) mit den genannten Quotienten multiplizieren.

Wie weiter vorne erörtert, sind jedoch die Wände der arteriellen Blutgefässe in Wirklichkeit elastisch dehnbar, so dass sich bei einem Druckanstieg zusätzlich zur Strömungsgeschwindigkeit des Blutes auch der Innenradius r sowie die Durchlassquerschnittsfläche der Blutgefässe vergrössert.

Wenn man ein Blutgefäss als Rohr mit einer elastisch dehnbaren Wand auffasst, ist die relative Dehnung epsilon des Umfangs sowie auch des Durchmessers gegeben durch die Formel

$$epsilon = [p \, (2 - mü) \, d^2] \, / \, E \, (D^2 - d^2) \qquad (8).$$

Dabei bezeichnet mü die Querkontraktionszahl der Blutgefässwand und D den Aussendurchmesser des Blutgefässes.

Die Dehnung eines Blutgefässes ist also vom Verhältnis zwischen dem Blutdruck p und dem Elastizitätsmodul E abhängig. Selbstverständlich ist umgekehrt auch die Dehnung und damit der Blutdruck vom Elastizitätsmodul abhängig. Zum Beispiel bewirkt eine Verkleinerung der Dehnbarkeit - bei konstant bleibender Förderleistung des Herzens - eine Vergrösserung des Blutdrucks. Der Innenradius r und Elastizitätsmodul E der Blutgefässwände hängen von der Art der beim Messen erfassten Blutgefässe, von der individuellen anatomischen Konfiguration der untersuchten Person und auch vom physiologischen Zustand der Blutgefässe ab. Zum Beispiel ist der Elastizitätsmodul bei einem jungen Sportler kleiner als bei einem alten, an Arteriosklerose leidenden Menschen. Zudem kann der Innenradius und/oder der Elastizitätsmodul während der Messzeitdauer relativ schnell ändern. Eine solche Änderung kann etwa durch eine Änderung der Spannung der zur Wand der Arterie gehörenden Muskelfasern verursacht werden. Die Spannung von zu arteriellen Blutgefässen gehörenden Muskelfasern kann zum Beispiel geändert werden, wenn für die Verdauung vorübergehend ein grösserer Teil des Blutstromes zu den Verdauungsorganen geleitet wird oder wenn die Durchblutung der Haut zur Anpassung an Änderungen der Umgebungstemperatur geändert wird. Des weitern kann die Spannung der besagten Muskelfasern auch durch die Tätigkeit und den Gemütszustand der untersuchten Person beeinflusst werden. Der Elastizitätsmodul E der Blutgefässwände ist jedoch gemäss der Formel (2) oder (3) mit der Pulswellengeschwindigkeit $c_{pw}$ verknüpft. Die Pulswellengeschwindigkeit oder eine daraus abgeleitete Grösse gibt daher ein Mass für den relativen Wert des Elastizitätsmoduls.

Wenn v ändert, ist also $r^2$ in der Formel (7) in Wirklichkeit abweichend von der vorherigen Annahme nicht konstant, sondern ändert ebenfalls. Da die zusätzlich zur Strömungsgeschwindigkeit v gemessene Pulswellengeschwindigkeit $c_{pw}$ gemäss der Formel (2) oder (3) ein Mass für den Elastizitätsmodul E gibt, kann der Einfluss der Elastizität beim Auswerten der Messwerte v und $c_{pw}$ durch die Auswertungsvorrichtung 65 berücksichtigt werden.

Es wurden Vergleichsmessungen durchgeführt, bei denen gleichzeitig mit der erfindungsgemässen Einrichtung die Strömungsgeschwindigkeit v sowie die Pulswellengeschwindigkeit $c_{pw}$ und mit der Eich-Messvorrichtung 71 nach der Methode von Riva-Rocci der Blutdruck gemessen wurde. Gemäss diesen Vergleichsmessungen besteht bei konstanter Pulswellengeschwindigkeit $c_{pw}$ eine sehr enge Korrelation zwischen dem Blutdruck p und der Strömungsgeschwindigkeit v sowie auch der Durcnflussmenge Q. Wenn hingegen die Pulswellengeschwindigkeit ändert, so ändert auch die Verknüpfung des Blutdrucks mit der Strömungsgeschwindigkeit.

Bei einer bestimmten, konstanten Pulswellengeschwindigkeit ist also jedem Wert von v in eindeutiger Weise ein Wert von p zugeordnet. Wenn die Pulswellengeschwindigkeit ändert, verursacht dies auch eine Änderung der Verknüpfung zwischen v und p. Dabei ergibt sich aber bei jedem beliebigen, aber gleichbleibenden Wert der Pulswellengeschwindigkeit eben eine eindeutige Verknüpfung zwischen v und p.

Wie bereits erwähnt, kann die Auswertungsvorrichtung 65 aus den ihr von der Erreger- und Detektorschaltung 61 in Form elektrischer Signale zugeführten Messwerten ein Mass für die momentanen Strömungsgeschwindigkeiten und/oder Durchflussmengen bei den beiden Sensoren 21, 23 gebende Werte und einen relativen Wert für die Pulswellengeschwindigkeit errechnen. Aus den beim Messen für die Pulswellengeschwindigkeit ermittelten Messwerten kann nötigenfalls ferner eine Grösse gebildet - d.h. berechnet - werden, die ein Mass für den Elastizitätsmodul gibt. Die Auswertungsvorrichtung 65 ist nun ausgebildet, um aus den eingegebenen Eichwerten gebildete Werte sowie Werte der beiden gemessenen Grössen - d.h. der Strömungsgeschwindigkeit und/oder Durchflussmenge und der Pulswellengeschwindigkeit - in einer vorgegebenen Weise derart miteinander zu verknüpfen, dass eine Grösse gebildet und durch ein elektrisches Signal dargestellt wird, die ein Mass für mindestens einen charakteristischen Wert des Blutdrucks und nämlich mindestens für den Wert des besonders wichtigen, systolischen Blutdruck $p_s$ gibt. Ferner erzeugt die Auswertungsvorrichtung 65 zum Beispiel auch noch elektrische Signale, die ein Mass für den zeitlich über eine Pulsperiodendauer gemittelten, mittleren Blutdruck $\bar{p}$ und/oder für den diastolischen Blutdruck $p_d$ geben. Zudem erzeugt die Auswertungsvorrichtung 65 vorzugsweise ein elektrisches Signal, das ein Mass für die Pulsfrequenz f gibt.

Die Auswertungsvorrichtung 65 kann den Blutdruck auf verschiedenartige Weise aus den Messwerten der Strömungsgeschwindigkeit und der Pulswellengeschwindigkeit ermitteln. Einige solche Möglichkeiten sollen nun erläutert werden. Der Blutdruck p kann zum Beispiel dargestellt werden durch die Formel

$$p = k_1 f_1 (v, c_{pw}) + p_1 \qquad (9)$$

Dabei ist $k_1$ und $p_1$ je eine Konstante und $f_1(v, c_{pw})$ eine von den Variablen v und $c_{pw}$ abhängige Funktion. Der Begriff "Funktion" wird hierbei sehr allgemein verstanden und soll in beliebiger Form festgelegte Zuordnungsvorschriften umfassen, die einem Paar von diskreten Werten oder Wertebereichen der unabhängigen Variablen v und $c_{pw}$ jeweils einen Funktionswert zuordnen. Man kann zum Beispiel aufgrund der genannten Vergleichsmessungen eine Tabelle erstellen, die jeden im vorgesehenen Messbereich liegenden Wert oder Wertebereich der Variablen v und $c_{pw}$ einen Wert der Funktion $f_1$ zuordnet. Diese Tabelle kann dann bei oder nach der Herstellung der Einrichtung in einem Speicher - beispielsweise einen ROM-Speicher - der Auswertungsvorrichtung gespeichert werden und bleibt darnach für alle durchzuführenden Messungen erhalten.

Da zum Eichen nur eine kurze Zeitdauer erforderlich ist, bleibt die Pulswellengeschwindigkeit während des Eichvorgangs normalerweise konstant. Man kann daher beim Eichen einerseits mit der Eich-Messvorrichtung 71 die Werte des systolischen und des diastolischen Blutdrucks und andererseits mit der erfindungsgemässen Einrichtung 11 mindestens annähernd gleichzeitig, beispielsweise beim gleichen Herzschlag, die diesen zwei Blutdruckwerten bei der gerade vorhandenen Pulswellengeschwindigkeit zugeordneten Werte der Strömungsgeschwindigkeit v messen. Die mit der Eich-Messvorrichtung 71 gemessenen Blutdruckwerte können mit der Eichwerteingabevorrichtung 63 der Auswertungsvorrichtung 65 zugeführt werden, welche die zwei Konstanten $k_1$ und $p_1$ bestimmt und dann bis zur nächsten Eichung in einem löschbaren Speicher speichert. Die Auswertungsvorrichtung kann des weitern ausgebildet und/oder programmiert sein, um den beim Messen gemessenen Werten von v und $c_{pw}$ aufgrund der gespeicherten Tabelle einen Wert der Funktion $f_1(v, c_{pw})$ zuzuordnen und aus diesem gemäss der Formel (9) den momentanen Wert des Blutdrucks berechnen.

Im übrigen wurde gefunden, dass die Funktion $f_1$ (v, $c_{pw}$) in guter Näherung in ein Produkt von zwei Funktionen $g_1(v)$ und $h_1(c_{pw})$ aufgespalten werden kann, von denen die erste nur von v und die zweite nur von $c_{pw}$ abhängig ist. Der Blutdruck kann also näherungsweise auch dargestellt werden durch die Formel

$$p = k_1\, g_1(v)\, h_1(c_{pw}) + p_1 \qquad (10)$$

Statt bei den Vergleichsmessungen die Werte der von zwei Variablen abhängigen Funktion $f_1$ zu ermitteln, und zu speichern, besteht daher auch die Möglichkeit, bei den Vergleichsmessungen die Werte der beiden Funktionen $g_1(v)$ und $h_1(c_{pw})$ für verschiedene Werte von v bzw. $c_{pw}$ zu bestimmen und in Form einer Tabelle zu speichern. Heim Durchführen einer Messung kann die Auswertungsvorrichtung dann die den Messwerten von v und $c_{pw}$ zugeordneten Werte der Funktionen $g_1(v)$ bzw. $h_1(c_{pw})$ in der gespeicherten Tabelle abrufen und gemäss der Formel (10) den Blutdruck p berechnen.

Des weitern besteht die Möglichkeit, aus den bei den Vergleichsmessungen ermittelten Daten durch ein mathematisches Näherungsverfahren - d.h. eine Ausgleichs- oder Regressionsrechnung - eine "konkrete" Funktion abzuleiten, welche die vorher nur abstrakt definierte Funktion $f_1$ oder mindestens eine der Funktionen $g_1$, $h_1$ darstellt. Unter einer "konkreten" wird hierbei eine Funktion oder Formel verstanden, die wie zum Beispiel eine Potenzreihe eine Rechenvorschrift zum Berechnen der Funktionswerte gibt. Der Mikroprozessor der Auswertungsvorrichtung kann dann derart programmiert werden, dass er aus den Messwerten von v und/oder $c_{pw}$ mit Hilfe der genannten Rechenvorschrift die Werte der betreffenden Funktion ausrechnen kann. Falls die Auswertung aufgrund der Formel (10) erfolgt, können entweder die Werte beider Funktionen $g_1$, $h_1$ gemäss einer Rechenvorschrift berechnet oder die Werte von einer der beiden Funktionen $f_1$, $g_1$ aus einer gespeicherten Tabelle abgerufen und nur die Werte der andern Funktion gemäss einer Rechenvorschrift berechnet werden. Wie schon erörtert, wäre p im Fall, dass die Blutgefässe starre Wände hätten, proportional zu v. Man kann daher eventuell als Werte für die Funktion $g_1(v)$ die Werte der Variablen v einsetzen und also in der Formel (10) $g_1(v)$ durch v ersetzen.

Des weitern besteht die Möglichkeit aufgrund der Theorie oder durch Kombinieren von theoretischen Ableitungen mit Ergebnissen von Vergleichsmessungen noch andere konkrete Formeln abzuleiten, die zwei durch Eichen bestimmte Konstanten erhalten. Selbstverständlich kann man auch versuchen, die Verknüpfung zwischen dem Blutdruck p und den Variablen v und $c_{pw}$ statt durch die Formeln (9) oder (10) durch eine Formel darzustellen, die nur eine einzige durch Eichen zu ermittelnde Konstante enthält.

Wie schon erwähnt, besteht andererseits die Möglichkeit, beim Eichen zusätzlich zu den Werten des systolischen und diastolischen Blutdrucks noch mindestens einen zwischen diesen Blutdruckwerten liegenden Zwischenwert zu messen und als Eichwert zu verwenden. Ferner kann dann mit der Einrichtung 11 auch die diesem bzw. jedem Blutdruck-Zwischenwert entsprechende Wert der Strömungsgeschwindigkeit v gemessen werden. Dies ermöglicht dann, die Verknüpfung des Blutdrucks p mit den gemessenen variablen Grössen v und $c_{pw}$ durch Formeln darzustellen, die drei oder noch mehr durch Eichen bestimmbare Konstanten enthalten.

Es sei hier noch angemerkt, dass die Auswertungsschaltung den diastolischen Blutdruck "direkt" aus den Eichwerten sowie den Messwerten oder "indirekt" aus dem allenfalls erfassten, mittleren Blutdruck ermitteln kann. Der zeitlich über eine Pulsperiodendauer gemittelte, mittlere Blutdruck $\bar{p}$ ist nämlich mit dem systolischen Blutdruck $p_s$ und dem diastolischen Blutdruck $p_d$ verknüpft. Gemäss experimentellen Untersuchungen kann die Verknüpfung dargestellt werden durch die Formel

$$\bar{p} = p_d + (p_s - p_d)\,/\,k \qquad (11).$$

Durch Umformung dieser Formel ergibt für den diastolischen Blutdruck die Formel

$$p_d = (k\,\bar{p} - p_s)\,/\,(k - 1) \qquad (12).$$

In diesen beiden Formeln ist k eine vom als Messbereich gewählten Körperbereich abhängige, aber mindestens annähernd zeitlich konstante Zahl, die für zentrale, d.h. entlang dem Strömungsweg beim Herzen liegende, arterielle Blutgefässe etwa den Wert 2 und für periphere, d.h. entlang dem Strömungsweg relativ weit vom Herzen entfernte, arterielle Blutgefässe etwa den Wert 3 hat. Für die grosse Arterie 3 des Arms hat k einen zwischen 2 und 3, aber nahe bei 2 liegenden Wert.

Bei der indirekten Methode werden zuerst der mittlere Blutdruck $\bar{p}$ und der systolische Blutdruck $p_s$ ermittelt. Danach wird aus $\bar{p}$ und $p_s$ mit der Formel (12) der diastolische Blutdruck berechnet. Der Wert der in den Formeln (11) und (12) enthaltenen Zahl k kann zum Beispiel - abhängig von der gewählten Messstelle - manuell eingegeben oder eventuell aus eingegebenen Blutdruck-Eichwerten und aus im Eichmodus von der Einrichtung 11 ermittelten Messwerten automatisch von der Auswertungsvorrichtung 65 bestimmt werden. Die "indirekte" Ermittlung des diastolischen Blutdrucks kann zum Beispiel eventuell vorteilhaft sein, um Verfälschungen des Messwertes des diastolischen Blutdrucks durch Nullpunktdriftvorgänge zu reduzieren.

Gemäss den Formeln (5) und (6) ist das Verhältnis zwischen der Durchflussmenge und dem Druck bei der Strömung durch ein starrwandiges Rohr und damit selbstverständlich auch bei der Strömung durch ein Blutgefäss auch von der Viskosität abhängig. Der Viskositätskoeffizient von Blut ändert jedoch normalerweise zeitlich höchstens geringfügig sowie langsam. Wenn die Einrichtung zum Beispiel ein Mal täglich geeicht wird, bleibt der Viskositätskoeffizient in den zwischen aufeinanderfolgenden Eichungen liegenden Messintervallen praktisch konstant. Der Viskositätskoeffizient kann jedoch eventuell durch gewisse medizinische Behandlungen verändert werden. Falls solche Behandlungen durchgeführt werden, kann vorgesehen werden, die Einrichtung 11 nach jeder derartigen Behandlung neu zu eichen. Wie noch erläutert wird, kann die Einrichtung zudem ausgebildet werden, um auch noch eine Grösse zu ermitteln, die ein Mass für die Viskosität und zum Beispiel für den relativen Wert des bereits erwähnten, dynamischen Viskositätskoeffizienten eta und/oder für den relativen Wert des kinematischen Viskositätskoeffizienten nü gibt.

Beim Messen zeigt die Anzeigevorrichtung 45 die ermittelten Werte des systolischen Blutdrucks $p_s$, des mittleren Blutdrucks $\bar{p}$ und/oder des diastolischen Blutdrucks $p_d$ sowie die Pulsfrequenz f und eventuell noch andere Werte an. Hier sei angemerkt, dass die Einrichtung 11 die verschiedenen Werte des Blutdrucks sowie eventuell auch den Wert der Pulsfrequenz bei jedem Herz- oder Pulsschlag neu ermitteln und anzeigen kann. Die Einrichtung 11 kann jedoch stattdessen die Werte von $p_s$, $\bar{p}$, $p_d$ und f über einige Herz- oder Pulsschläge zeitlich mitteln und diese zeitlich gemittelten Werte anzeigen. Die Anzeigevorrichtung 45 kann ausgebildet sein, um mehrere Werte - zum Beispiel $p_s$, $\bar{p}$, $p_d$, f - gleichzeitig und fortlaufend anzuzeigen. Es kann jedoch auch vorgesehen sein, dass die Anzeigevorrichtung die verschiedenen anzeigbaren Werte oder einen Teil von diesen nur abwechselnd anzeigt. Im letzteren Fall kann zum Beispiel durch Betätigen von mindestens einem der Schalt- und/oder Stellorgane gewählt werden, welche von mehreren möglichen Grössen angezeigt werden soll.

Die Schalt- und/oder Stellorgane 47 ermöglichen auch das Einstellen mindestens eines Grenzwerts. Die Einrichtung 11 kann beispielsweise ausgebildet sein, um einer Bedienungsperson die Eingabe von je mindestens einem untern und einem obern Grenzwert für den systolischen Blutdruck und für die Pulsfrequenz zu ermöglichen. Die Auswertungsvorrichtung 65 der Einrichtung 11 kann beim Messen dann die fortlaufend neu ermittelten Werte von $p_s$ und f mit den vorgegebenen Grenzwerten vergleichen und beim Überschreiten oder Unterschreiten eines Grenzwertes ein entsprechendes elektrisches Signal erzeugen und der Alarmiervorrichtung 51 und gegebenenfalls der eventuell zusätzlich noch vorhandenen, zentralen Überwachungs- und Alarmiervorrichtung zuführen. Das Über- oder Unterschreiten des betreffenden Grenzwerts wird dann durch einen zugeordneten optischen Alarmsignalgeber 53 der Alarmiervorrichtung 51 und allenfalls sonst noch vorhandene optische und/oder akustische Alarmsignalgeber signalisiert. Ferner können die für den Blutdruck und die Pulsfrequenz ermittelten Werte gespeichert und/oder registriert werden.

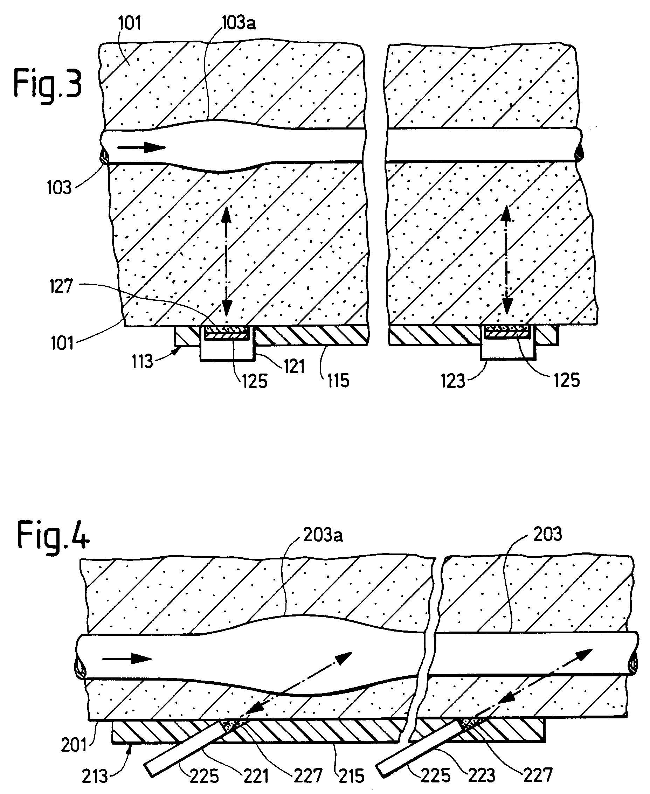

In der Figur 3 ist ein Glied 101 mit einer grossen Arterie 103 ersichtlich, die eine durch das pulsierend fliessende Blut verursachte, mit der Pulswellengeschwindigkeit fortschreitende Erweiterung 103a aufweist. Die gemäss der Figur 3 aussen am Glied 101 angeordneten Sensormittel 113 weisen einen lösbar am Glied 101 befestigten Halter 115, einen ersten Sensor 121 und einen zweiten Sensor 123 auf. Die beiden Sensoren 121, 123 sind analog zu den Sensoren 21 bzw. 23 entlang dem Glied gegeneinander versetzt, weisen jedoch anstelle optoelektronischer Wandler je mindestens einen Ultraschall-Wandler 125 auf. Der Halter 115 kann in einem zur Längsrichtung des Arms 101 rechtwinkligen Schnitt auf seiner zum Anliegen an diesem bestimmten Seite eben und gerade oder leicht gebogen sein. Ferner kann der Halter eventuell ein wenig flexibel sein, so dass er möglichst gut passend an der Oberfläche des Arms 101 anliegen kann. Jeder Ultraschall-Wandler 125 weist mindestens ein piezoelektrisches Element auf und ist derart ausgebildet, dass er als Strahler und als Empfänger für Ultraschallwellen dienen und also sowohl elektrische Signale - nämlich Spannungsimpulse - in Ultraschallwellen als auch zurückgestreute, empfangene Ultraschallwellen in elektrische Signale umwandeln kann. Die Wandler und ihre in der Figur 3 durch Doppelpfeile angedeuteten Strahlungs/Empfangs-Hauptrichtungen und/oder Mittelachsen sind annähernd rechtwinklig zur Längsrichtungen des Arms 101 und der Arterie 103. Zwischen den dem Arm 101 zugewandten Seiten der piezoelektrischen Elemente und der Oberfläche des Arms ist ein Ultraschall-Übertrager 127 vorhanden, der zum Beispiel aus einer gallertartigen Masse aus Polyäthylenglykol besteht.

Die Ultraschall-Wandler 125 der Sensoren 121, 123 sind elektrisch leitend mit einer Speise- und Detektorvorrichtung der elektronischen Schaltungsmittel eines nicht gezeichneten Anzeige- und Überwachungsgerätes verbunden. Die Speise- und Detektorvorrichtung ist derart ausgebildet, dass die Ultraschall-Wandler impulsweise Ultraschallwellen in den Arm einstrahlen und zwischen den aufeinanderfolgenden Impulsen reflektierte bzw. zurückgestreute Ultraschallwellen empfangen. Die Wandler werden derart am Arm 101 angeordnet, dass die von ihnen erzeugten Ultraschallwellenbündel die Arterie 103 kreuzen. Die in die grosse Arterie 103 eindringenden Ultraschallwellen werden von dem durch die Arterie 103 fliessenden Blut mindestens zum Teil gestreut und/oder reflektiert. Die elektronischen Schaltungsmittel des nicht gezeichneten Anzeige-und Überwachungsgerätes sind ausgebildet, um aus den beim Empfang von zurückgestreutem Ultraschall von den Sensoren 121, 123 erzeugten elektrischen Signalen eine Grösse zu erzeugen, die ein Mass für Strömungsgeschwindigkeit des Blutes gibt. Die Bestimmung der Strömungsgeschwindigkeit kann dabei zum Beispiel gleich oder ähnlich erfolgen, wie aus den in der Einleitung zitierten Publikationen für ein quer zur Strömungsrichtung gerichtetes Ultraschallwellenbündel verwendende Methode bekannt ist.

Die restlichen Teile der die Sensormittel 113 aufweisenden Einrichtung können - soweit vorgängig nichts anderes angegeben wurde - ähnlich ausgebildet sein und ähnliche Funktionen ausüben, wie es für die Einrichtung 11 beschrieben wurde.

In der Figur 4 ist ein Arm 201 mit einer grossen Arterie 203 ersichtlich, die eine fortschreitende Erweiterung 203a aufweist. Die in der Figur 4 ebenfalls ersichtlichen Sensormittel 213 weisen einen lösbar am Arm befestigten Halter 215 auf. An diesen sind zwei Sensoren 121, 123 befestigt, von denen jeder mindestens einen Ultraschall-Wandler 125 mit einem piezoelektrischen Element besitzt und sowohl als Ultraschallstrahler zum impulsweisen Anstrahlen von Ultraschall als auch als Ultraschallempfänger dient. Die Ultraschall-Wandler 225 sind abweichend von den Ultraschall-Wandlern 125 derart angeordnet, dass ihre Strahlungs/Empfangs-Hauptrichtungen und/oder -Mittelachsen zur Längsrichtung des Arms 201 und der grossen Arterie 203 geneigt sind, und dementsprechend eine zur Strömungsrichtung des durch die grosse Arterie 203 fliessenden Blutes parallele Komponente haben. Zwischen der Oberfläche des Arms 201 und den diesem zugewandten Seiten oder Enden der Wandler 225 ist ein Ultraschall-Übertrager 227 vorhanden, der wiederum aus einer Polyäthylenglykol-Masse bestehen kann.

Die Sensoren sind mit einer nicht gezeichneten Anzeige-und Überwachungsvorrichtung verbunden, deren elektronische Schaltungsmittel ausgebildet sind, um die Strömungsgeschwindigkeit aufgrund des Dopplereffekts zu ermitteln.

Die Viskosität des Blutes wird vor allem durch die Anzahl, Grösse und Struktur der in einer Volumeneinheit des Blutes enthaltenen, roten und eventuell weissen Blutkörperchen bestimmt. Licht- und Ultraschallwellen werden im Blut vor allem durch dessen Blutkörperchen gestreut. Aus der Streuung von Licht- und vor allem von Ultraschallwellen kann daher eine Grösse abgeleitet werden, die ein Mass für den dynamischen und/oder kinematischen Viskositätskoeffizienten und/oder mindestens für dessen zeitliche Änderung gibt. Dementsprechend können die zur Erfassung von gestreutem Licht oder Ultraschall ausgebildeten Einrichtungen - wie schon erwähnt - mit Mitteln versehen und ausgebildet werden, um eben aufgrund der Streuung noch eine Grösse zu ermitteln, die ein Mass für die Viskosität des Blutes oder mindestens für deren Änderung gilt.

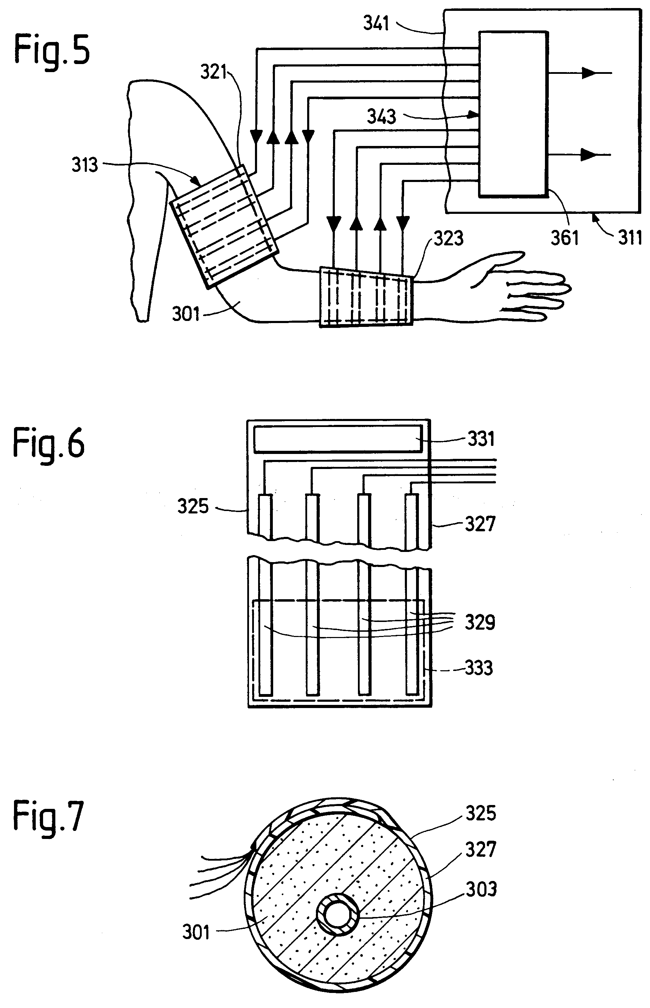

In den Figuren 5 und 7 ist ein Arm 301 mit einer grossen Arterie 303 gezeichnet. Die ebenfalls in der Figur 5 ersichtliche Einrichtung 311 ist zur Ermittlung des Blutdrucks durch Erfassen der elektrischen Impedanz oder Admittanz ausgebildet. Die Einrichtung 311 besitzt Sensormittel 313 mit zwei lösbar am Arm 301 befestigte Sensoren 321, 323, die wiederum entlang dem Arm in Abstand voneinander stehen. Dabei ist zum Beispiel der eine, erste Sensor 321 am Oberarm und der andere, zweite Sensor 323 am Unterarm befestigt. Jeder der beiden Sensoren, von denen einer auch in den Figuren 6 und 7 ersichtlich ist, besitzt eine Manschette 325 mit einem flexiblen, elektrisch isolierenden und eventuell ein wenig elastischen Band 327, das auf seiner bei der Benutzung dem Arm zugewandten Seite mit vier Kontakt-Elektroden 329 versehen ist, die aus flexiblen, elektrisch leitenden, in Abstand nebeneinander parallel zur Band-Längsrichtung verlaufenden Metallfolienstreifen bestehen. Am Band 327 sind Klettverschlusselemente 331, 333 befestigt. Für die Durchführung einer Messung wird die Manschette 325 jedes Sensors derart um den Arm 301 herumgewickelt, dass die Kontakt-Elektroden 329 an diesem anliegen und diesen vollständig umschliessen. Die Manschette wird dabei bei ihren sich überlappenden Endabschnitten mit Hilfe der Klettverschlusselemente 331, 333 befestigt.

Eine Anzeige- und Überwachungsvorrichtung 341 besitzt elektronische Schaltungsmittel 343. Jede Kontakt-Elektrode 329 ist durch einen Leiter eines Kabels mit einer zu den Schaltungsmitteln 343 gehörenden Speise- und Detektorvorrichtung 361 verbunden. Die letztere weist einen Generator oder zwei Generatoren auf, um eine Speisespannung bzw. einen Speisestrom mit einer 80 kHz bis 100 kHz betragenden Frequenz zu erzeugen und den beiden äussersten Elektroden der beiden Sensoren 321,323 zuzuführen. Der bzw. jeder Generator kann ausgebildet sein, um bei jedem Sensor einen Strom mit konstanter Amplitude in den Arm 301 einzuspeisen. Die Speise- und Detektorvorrichtung 361 ist des weitern ausgebildet, um aus den zwischen den beiden innern Elektroden jedes Sensors abnehmbaren elektrischen Spannungen bei jedem Sensor die elektrische Impedanz oder Admittanz zu erfassen. Die Vorrichtung 361 kann ferner aus dem zeitlich im Takt des Pulses ändernden Teil der Impedanz bzw. Admittanz mindestens eine erste Grösse bilden, die ein Mass für das Volumen gibt. Das Volumen des Blutes in einem Blutgefäss ist proportional zu dessen Durchlassquerschnittsfläche und proportional zum Quadrat des Innendurchmessers des Blutgefässes. Bei der Pulsation der Blutströmung ändern vor allem die Volumina, Durchlassquerschnittsflächen und Durchmesser der grossen Arterien - d.h. der Arteria brachialis des Oberarms und der Arteria radialis sowie der etwas kleineren Arteria ulnaris des Unterarms. Die Messung der Impedanz oder Admittanz gibt also auch ein Mass für die Durchlassquerschnittsfläche und für den Innendurchmesser von mindestens einer im Messbereich vorhandenen Arterie. Die elektronischen Schaltungsmittel der Vorrichtung 341 können ferner ausgebildet sein, um durch einen elektrisch in analoger oder digitaler Form durchgeführten Differenziervorgang der ersten Ableitung des Volumens nach der Zeit zu bilden. Die für die beiden Messbereiche ermittelten Ableitungen oder Differentialquotienten geben dann je ein Mass für die Durchflussmenge des arteriellen Blutes in den von den beiden Sensoren erfassten Armbereichen. Die Einrichtung 311 ermöglicht also bei den zwei Messbereichen eine erste Grösse zu ermitteln, die ein relatives Mass für das Volumen und/oder die Durchflussmenge des Blutes und/oder für die Durchlassquerschnittsfläche und/oder den Innendurchmesser mindestens eines Blutgefässes gibt. Für weitere allgemeine Grundlagen zu dieser Messart sei auf die in der Einleitung zitierten Publikationen verwiesen.

Die Speise- und Detektorvorrichtung 361 ist mit einer nicht gezeichneten Auswertungsvorrichtung verbunden, die ähnlich wie die Auswertungsvorrichtung 65 aus der zeitlichen Verschiebung der mit den beiden Sensoren erfassten, pulsierenden, ersten Grösse die Pulswellengeschwindigkeit ermitteln kann. Die zu den elektronischen Schaltungsmitteln 343 gehörende Auswertungsvorrichtung kann zudem ausgebildet sein, um den Blutdruck aus den bei mindestens einem Messbereich für mindestens eine der als erste Grösse gemessenen Varianten gemessenen Messwerten der Pulswellengeschwindigkeit $c_{pw}$ und den Eichwerten in analoger Weise zu ermitteln, wie die Auswertungsvorrichtung 65 den Blutdruck aus v und $c_{pw}$ ermittelt. Im Fall, dass als erste Grösse die Durchflussmenge oder die dazu proportionale Durchlassquerschnittsfläche ermittelt und ferner angenommen wird, dass die Blutgefässe starre Wände hätten, wäre übrigens der Blutdruck gemäss der Formel (5) proportional zum Verhältnis $Q/r^4$.

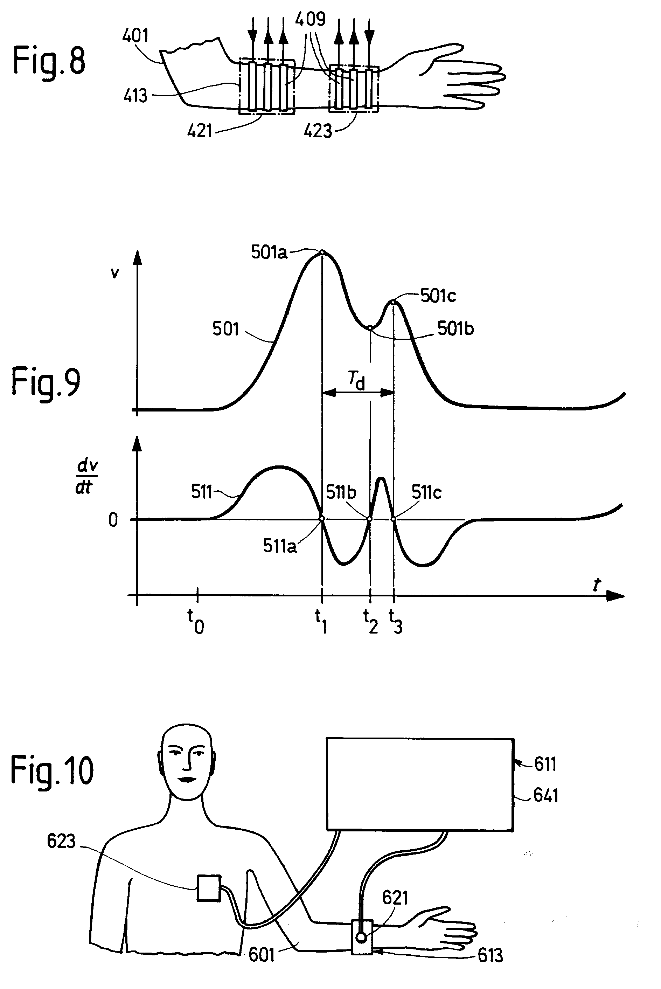

An dem in der Figur 8 ersichtlichen Arm 401 sind Sensormittel 413 lösbar befestigt, die zwei Sensoren 421, 423 zur Messung der elektrischen Impedanz oder Admittanz besitzen. Die Sensormittel 413 unterscheiden sich von den Sensormitteln 313 dadurch, dass sie insgesamt nur sechs Kontakt-Elektroden 409 besitzen. Beim Messen wird bei den beiden äussersten Elektroden 409 ein für beide Sensoren gemeinsamer, hochfrequenter Wechselstrom in den Arm eingeleitet. Im übrigen besitzen die beiden Sensoren je eine strichpunktiert angedeutete Manschette, könnten aber auch eine gemeinsame Manschette aufweisen.

Die anhand der Figuren 5 bis 8 beschriebenen Einrichtungen können eventuell noch dadurch geändert werden, dass einige oder alle Kontakt-Elektroden durch elektrisch gegen die Körperoberfläche isolierte Elektroden ersetzt werden, die den Arm oder einen andern Körperteil umschliessen. Jede dieser elektrisch isoliert am Körper angebrachten Elektroden bildet dann gewissermassen eine spulenartige Antenne, welche ebenfalls eine Messung der Impedanz oder Admittanz ermöglicht.

Wie schon in der Einleitung erwähnt, könnte die Einrichtung auch ausgebildet sein, um als erste Grösse den Durchmesser einer Arterie, und zwar vorzugsweise einer grossen Arterie, unter Verwendung der Ultraschallreflexion zu messen. Die Einrichtung kann in diesem Fall beispielsweise Sensoren aufweisen, die gleich wie die Sensoren 121, 123 mit Ultraschall-Wandlern 125 versehen sind. Die elektronischen Schaltungsmittel können dann ausgebildet sein, um aufgrund der Reflexion von Ultraschallwellen an mindestens einem Abschnitt der Arterienwand und vorzugsweise an zwei sich ungefähr diametral gegenüberstehenden Abschnitten der Arterienwand elektrische Signale bilden, die ein Mass für die momentanen Werte und/oder zeitlichen Änderungen der inneren oder äusseren oder mittleren Durchmesser oder anderer Querschnittsabmessungen und damit auch der Durchlassquerschnittsflächen bei den beiden Sensoren geben. Aus der zeitlichen Verschiebung zwischen den sich bei den beiden Sensoren ergebenden, periodischen, zum Puls synchronen, zeitlichen Änderungen kann dann die Pulswellengeschwindigkeit ermittelt werden. Ferner kann aus den gemessenen Grössen und eingegebenen Eichwerten der Blutdruck ermittelt werden. Dies kann auf ähnliche Weise geschehen, wie es für die anhand der Figuren 1 und 2 beschriebene, die Strömungsgeschwindigkeit messende Einrichtung 11 erläutert wurde.

Bei den bisher beschriebenen Ausführungsformen der erfindungsgemässen Einrichtung sind zwei gleichartige, entlang einer Arterie versetzte Sensoren vorhanden. Wie schon in der Einleitung erörtert, kann man jedoch auch Sensormittel vorsehen, die nur einen einzigen Sensor zum Messen der ersten Grösse aufweisen.

Die Pulswellengeschwindigkeit kann dann beispielsweise - wie ebenfalls in der Einleitung dargelegt - aus der durch die Reflexion der Pulswellen verursachten Beeinflussung des zeitlichen Verlaufs der als erste Grösse gemessenen Variablen ermittelt werden. Dies soll nun anhand der Figur 9 für den Fall erläutert werden, dass ein Sensor und elektronische Schaltungsmittel vorhanden sind, um als erste Grösse mit Hilfe von Licht oder Ultraschall die Strömungsgeschwindigkeit v des Blutes messen. Die Figur 9 enthält zwei Teil-Diagramme mit einer gemeinsamen Abszisse, auf welcher die Zeit t aufgetragen ist. Im oberen Teil-Diagramm ist auf der Ordinate die Strömungsgeschwindigkeit v aufgetragen. Die Kurve 501 des oberen Teil-Diagramms zeigt den etwas schematisierten Verlauf der Strömungsgeschwindigkeit des eine Arterie durchströmenden Blutes während ungefähr einer Pulsperiode . Im unteren Teil-Diagramm ist auf der Ordinate der Differentialquotient $dv/dt$ aufgetragen. Die Kurve 511 des unteren Teil-Diagramms zeigt dementsprechend - ebenfalls schematisiert - den Verlauf des genannten Differentialquotienten. Gemäss der Kurve 501 beginnt die Strömungsgeschwindigkeit v bei einem Herzschlag ausgehend von einem Minimalwert im Zeitpunkt $t_0$ anzusteigen und erreicht im Zeitpunkt $t_1$, der mindestens ungefähr mit dem Auftreten des systolischen Blutdrucks zusammenfällt, ein Haupt-Maximum 501a. Die Strömungsgeschwindigkeit v fällt dann ab, erreicht im Zeitpunkt $t_2$ ein relatives Zwischen-Minimum 501b, steigt anschliessend wieder an und erreicht im Zeitpunkt $t_3$, in welchem bei der Blutdruckkurve der dikrotische Knoten auftritt, ein Neben-Maximum 501c. Daraufhin fällt die Strömungsgeschwindigkeit wieder bis etwa auf den anfänglichen Minimalwert ab. Die den Verlauf des Differentialquotienten $dv/dt$ darstellenden Kurve 511 hat dementsprechend in den Zeitpunkten $t_1$, $t_2$, $t_3$ je einen Nulldurchgang 511a bzw. 511b bzw. 511c, wobei sie die Null-Linie bei den Nulldurchgängen 511a und 511c von oben nach unten und beim Nulldurchgang 511b von unten nach oben durchdringt.

Wie in der Einleitung erörtert, wird der dikrotische Knoten und damit das diesem zugeordnete Neben-Maximum 501c durch die Interferenz der primären, vom Herzen weg verlaufenden Pulswelle mit einer reflektierten Pulswelle verursacht. Die Zeitdifferenz $T_d$ zwischen den beiden Maxima 501a und 501c gibt daher ein Mass für die Laufzeit der reflektierten Pulswellen und damit für die Pulswellengeschwindigkeit.

Die elektronischen Schaltungsmittel und insbesondere die Auswertungsvorrichtung der zur Ermittlung der Pulswellengeschwindigkeit aufgrund der Pulswellenreflexion vorgesehenen Einrichtung sind ausgebildet, um von der als erste Grösse gemessenen Strömungsgeschwindigkeit die erste Ableitung nach der Zeit - d.h. den Differentialquotienten dv/dt - und vorzugsweise auch noch die zweite Ableitung nach der Zeit, d.h. den Differentialquotienten $d^2v/dt^2$ zu bilden. Diese Differentiation kann elektrisch mit Hilfe einer Differenzierschaltung in analoger Form oder mit Hilfe eines Mikroprozessrechners in digitaler Form durchgeführt werden. Die Schaltungsmittel können dann die Zeitpunkte $t_1$ und $t_3$ aufgrund der Differentialquotienten identifizieren und die Zeitdifferenz $T_d$ messen. Die Zeitdifferenz $T_d$ oder eine damit verknüpfte Grösse, beispielsweise ihr Reziprokwert, bildet dann die ein Mass für die Pulswellengeschwindigkeit gebende, zweite Grösse, deren Wert in Form eines digitalen oder analogen, elektrischen Signals dargestellt wird.

Falls als erste Grösse anstelle der Strömungsgeschwindigkeit v eine andere der in Frage kommenden Variablen gemessen wird, kann die ein Mass für die Pulswellengeschwindigkeit gebende Grösse in ähnlicher Weise ermittelt werden, wie es anhand der Figur 9 für die Strömungsgeschwindigkeit v erläutert wurde.

In die Figur 10 sind eine Person mit einem Arm 601 und eine hier mit 611 bezeichnete Einrichtung zur Messung des Blutdrucks ersichtlich. Die Sensormittel 613 der Einrichtung 611 weisen nur einen einzigen, lösbar am Arm 601 befestigten Sensor 621 auf. Dieser besitzt beispielsweise zur Messung der Strömungsgeschwindigkeit mittels Licht oder Ultraschall dienende Wandler, könnte aber auch ausgebildet sein, um eine als erste Grösse verwendbare Variable durch eine andere Messung, etwa eine Impedanz- oder Admittanz-Messung zu ermitteln. Die Sensormittel 613 weisen jedoch noch etwa an der Brust der untersuchten Person befestigte, zusammen mindestens einen Sensor 623 bildende Kontakt-Elektroden auf. Der Sensor 621 und die Kontakt-Elektroden des Sensors 623 sind über Kabel mit der Anzeige- und Überwachungsvorrichtung 641 verbunden. Die elektronischen Schaltungsmittel von dieser sind ausgebildet, um zusammen mit den Kontakt-Elektroden des Sensors 623 analog wie bei der Elektrokardiographie mindestens einen Strom des Herzmuskels zu erfassen und die sogenannte R-Zacke des Kardiogramms zu ermitteln, bei welcher die Systole - d.h. die Kontraktion des Herzmuskels - und der Blutausstoss aus dem Herzen erfolgt. Die Vorrichtung 641 kann dann ferner die Zeitdifferenz zwischen der R-Zacke und dem Zeitpunkt ermitteln, in dem die Strömungsgeschwindigkeit des Blutes im Arm 601 oder die sonstige vom Sensor 621 gemessene, erste Grösse während einer Pulsperiode den Maximalwert erreicht. Diese Zeitdifferenz und deren Änderung oder eine aus dieser Zeitdifferenz abgeleitete Grösse und deren Änderung geben dann ein Mass für die Pulswellengeschwindigkeit und deren Änderung.

Die Einrichtungen und die Verfahren zu ihrem Betrieb können noch in anderer Hinsicht modifiziert werden. Dabei können insbesondere Merkmale von verschiedenen beschriebenen Einrichtungen bzw. Verfahren miteinander kombiniert werden. Zum Beispiel können Sensoren vorgesehen werden, die wie die Sensoren 21, 23 Lichtstrahler und Lichtempfänger aufweisen, aber analog wie die mit einem Ultraschall-Wandler versehenen Sensoren 121, 123 angeordnet sind, um Licht in einer zum Arm geneigten Richtung in diesen einzustrahlen, so dass dann eine optische Dopplereffekt-Messung durchgeführt werden kann.

Wenn eine Messung mit einem eine grosse Arterie kreuzenden Licht- oder Ultraschallstrahlenbündel durchgeführt werden soll, kann man ferner Sensoren vorsehen, von denen jeder mehrere gleichartige Wandler aufweist, die quer zur besagten Arterie gegeneinander versetzt sind. Wenn ein derartiger Sensor etwa an einen die genannte grosse Arterie enthaltenden Arm befestigt wird, kann ohne genaues Positionieren des Sensors erreicht werden, dass die Strahlungs-Mittelachse von einem seiner Wandler die grosse Arterie kreuzt. Die elektronischen Schaltungsmittel können dann ausgebildet sein, um beim Messen automatisch das Signal von demjenigen Wandler für den die Weiterverarbeitung auszuwählen, das am stärksten durch den Puls moduliert ist.

Die beschriebenen, erfindungsgemässen Einrichtungen und Verfahren haben alle den Vorteil, dass eine langdauernde, kontinuierliche oder mindestens quasi-kontinuierliche, zum Beispiel bei jedem Herzschlag stattfindende Messung des Blutdrucks möglich ist, wobei die untersuchte Person oder eventuell das untersuchte Tier nur relativ wenig gestört wird. Dabei ist es insbesondere von Vorteil, dass nur gerade zum Eichen eine einmalige Eich-Messung oder einige wenige Eich-Messungen nach der Methode von Riva-Rocci durchgeführt werden muss bzw. müssen und dass während des grössten Teils der Messdauer kein Aufblasen und Entlüften einer Manschette erforderlich ist.

Das unter Verwendung von einer der beschriebenen Einrichtungen durchgeführte Verfahren zur Blutdruckmessung und zur Pulsfrequenzmessung kann in der Therapie für langdauernde Überwachung und ferner zum Beispiel noch bei Untersuchungen gewerblich angewendet werden, die in Spitälern und eventuell Arztpraxen für die Abklärung des allgemeinen Gesundheitszustandes, für die humanmedizinische sowie die tiermedizinische Forschung und bei der Entwicklung sowie Prüfung von Heilmitteln durchgeführt werden.

**Patentansprüche**

1. Einrichtung zur Blutdruckmessung, mit Sensormitteln (13, 113, 213, 313, 413, 613) zum Anbringen an einem lebenden Körper und mit elektronischen Schaltungsmitteln (43, 343), die mit den Sensormitteln (13, 113, 213, 313, 413, 613) verbunden sind, wobei die Sensormittel (13, 113, 213, 313, 413, 613) und Schaltungsmittel (43, 343) ausgebildet sind, um in einem zumindest ein arterielles Blutgefäß (3, 4, 5, 103, 203, 303) enthaltenden Meßbereich zumindest eine sich zeitlich im Takt des Pulses ändernde erste Größe zu ermitteln, die ein Maß für die Strömungsgeschwindigkeit, die Durchflußmenge, das Volumen des arteriellen Blutes oder der Durchlaßquerschnittsfläche mindestens eines arteriellen Blutgefäßes (3, 103, 203, 303) und/oder deren Änderung gibt, und um eine zweite Größe zu ermitteln, die ein Maß für die Pulswellengeschwindigkeit und/oder deren Änderung gibt, wobei ferner die Schaltungsmittel (43, 343) ausgebildet sind, um durch Verknüpfen der beiden Größen unter Einbeziehung von Eichwerten mindestens eine Größe zu bilden, die ein Maß für den Blutdruck und/oder dessen Änderung gibt.

2. Einrichtung nach Anspruch 1, **dadurch gekennzeichnet,** daß die Sensormittel (13, 113, 213, 313, 413, 613) mindestens einen Sensor (21, 23, 121, 123, 221, 223) aufweisen, um Schall- oder Lichtwellen in den zumindest ein arterielles Blutgefäß (3, 4, 5, 103, 203) enthaltenden Meßbereich des Körpers einzustrahlen und um von arteriellem Blut gestreute Schall- oder Lichtwellen zu empfangen, und daß die Schaltungsmittel (43) ausgebildet sind, um aus den gestreuten, von mindestens einem Sensor (21, 23, 121, 123, 221, 223) aufgenommenen Schall- oder Lichtwellen als erstgenannte Größe die Strömungsgeschwindigkeit und/oder Durchflußmenge von den Meßbereich durchströmendem Blut zu ermitteln.

3. Einrichtung nach Anspruch 2, **dadurch gekennzeichnet**, daß die Sensormittel (13) ausgebildet sind, um Lichtwellen in den mindestens einen Meßbereich des Körpers einzustrahlen und zurückgestreute Lichtwellen zu empfangen sowie in mindestens ein elektrisches Signal umzuwandeln.

4. Einrichtung nach Anspruch 3, **dadurch gekennzeichnet**, daß mindestens ein Lichtstrahler (27) zur Erzeugung von Lichtwellen vorhanden ist, deren Wellenlänge mindestens 700 nm, höchstens 1200 nm und beispielsweise 800 nm bis 1000 nm beträgt.

5. Einrichtung nach Anspruch 2, **dadurch gekennzeichnet**, daß die Sensormittel (113, 213) mindestens einen Ultraschall-Wandler (125, 225) aufweisen, um Ultraschallwellen in den mindestens einen Meßbereich des Körpers einzustrahlen und zurückgestreute Ultraschallwellen zu empfangen und in mindestens ein elektrisches Signal umzuwandeln.

6. Einrichtung nach Anspruch 1 oder 2, **dadurch gekennzeichnet**, daß die Sensormittel (313, 413) Elektroden (329, 429) aufweisen und daß die Schaltungsmittel (343) ausgebildet sind, um die elektrische Impedanz oder Admittanz oder die zeitliche Änderung der Impedanz bzw. Admittanz von dem mindestens einen Meßbereich des Körpers zu ermitteln und daraus als erstgenannte Größe eine Größe zu bilden, die ein Maß für das Volumen und/oder die Durchflußmenge von im Meßbereich vorhandenem, arteriellem Blut und/oder für die Durchlaßquerschnittsfläche und/oder den Innendurchmesser von mindestens einem im Meßbereich vorhandenen Blutgefäß gibt.

7. Einrichtung nach Anspruch 1 oder 2, **dadurch gekennzeichnet**, daß die Sensormittel (113) mindestens einen Ultraschall-Wandler (125) aufweisen, um Ultraschallwellen in den mindestens einen Meßbereich des Körpers einzustrahlen und um von mindestens einem Wandabschnitt einer Arterie (103) reflektierte Ultraschallwellen zu empfangen, und daß die Schaltungsmittel ausgebildet sind, um als erstgenannte Größe eine Größe zu ermitteln, die ein Maß für den Durchmesser und/oder die Durchlaßquerschnittsfläche und/oder für die Änderung des Durchmessers und/oder der Durchlaßquerschnittsfläche der Arterie (103) gibt.

8. Einrichtung nach einem der Ansprüche 1 bis 7, **dadurch gekennzeichnet**, daß die Sensormittel (13, 113, 213, 313, 413) zwei Sensoren (21, 23, 121, 123, 221, 223, 321, 323, 421, 423) aufweisen, um bei zwei entlang einer Arterie (3, 103, 203, 303) voneinander in Abstand stehenden Meßbereichen die periodisch im Takt des Pules stattfindenden, zeitlichen Änderungen der die erstgenannte Größe bildenden Variablen zu ermitteln, und daß die Schaltungsmittel (43, 343) ausgebildet sind, um aus der zeitlichen Verschiebung zwischen den bei den beiden Sensoren (21, 13, 121, 123) ermittelten, periodischen, zeitlichen Änderungen der besagten Variablen die ein Maß für die Pulswellengeschwindigkeit gebende Größe zu ermitteln.

9. Einrichtung nach einem der Ansprüche 1 bis 7, **dadurch gekennzeichnet**, daß die Schaltungsmittel ausgebildet sind, um ein während einer Pulsperiode auftretendes, dem systolischen Blutdruck entsprechendes Haupt- Extremum (501a) sowie ein durch die Interferenz einer vom Herzen wegverlaufenden Pulswelle mit einer reflektierten Pulswelle verursachtes, zusätzliches Extremum zu identifizieren und die zeitliche oder phasenmäßige Verschiebung zwischen den beiden Extrema (501a, 501c) zu ermitteln sowie daraus die ein Maß für die Pulswellengeschwindigkeit gebende Größe zu bilden.

10. Einrichtung nach einem der Ansprüche 1 bis 7, **dadurch gekennzeichnet**, daß die Sensormittel (613) und Schaltungsmittel ausgebildet sind, um zusätzlich zur bei dem zumindest einen Meßbereich des Körpers als erstgenannte Größe ermittelten, periodisch ändernden Variablen noch einen elektrischen Strom des Herzmuskels zu ermitteln und aus der zeitlichen Verschiebung zwischen einer vorgegebenen, in jeder Pulsperiode auftretenden Stelle im zeitlich änderndem Verlauf des elektrischen Stroms des Herzmuskels und einer vorgegebenen, in jeder Pulsperiode auftretenden Stelle im zeitlichen Verlauf der als erstgenannte Größe ermittelten Variablen die ein Maß für die Pulswellengeschwindigkeit und/oder deren Änderung gebende Größe zu bilden, wobei die vorgegebenen Stellen zum Beispiel durch die R-Zacke der den zeitlichen Verlauf des Stroms darstellenden Kurve bzw. das dem systolischen Druck zugeordnete Extremum der als erstgenannte Größe ermittelten Variablen gebildet sind.

11. Einrichtung nach einem der Ansprüche 1 bis 10, **dadurch gekennzeichnet**, daß die Schaltungsmittel (43, 343) ausgebildet sind, um beim Messen jeweils einen gemessenen Wert der erstgenannten Größe und einen gemessenen Wert der ein Maß für die Pulswellengeschwindigkeit gegebenen Größe zum Ermitteln eines Wertes des Blutdruckes gemäß einer vorgegebenen Vorschrift, zum Beispiel durch die Zuordnung eines gespeicherten Funktionswertes zu mindestens einem der beiden gemessenen Werte und/oder zum Beispiel durch die Berechnung eines Funktionswertes aus mindestens einem der gemessenen Werte festlegt.

12. Einrichtung nach einem der Ansprüche 1 bis 11, **dadurch gekennzeichnet**, daß mindestens eine Eichwerteingabevorrichtung (63) vorhanden ist und daß die Schaltungsmittel (43, 343) ausgebildet sind, um nach der Eingabe mindestens eines Eichwertes, der durch eine am Körper durchgeführten Messung mit einer Eich-Meßvorrichtung (71) ermittelt wurde, den Blutdruck zumindest quasi-kontinuierlich, beispielsweise bei jedem Herzschlag, zu bestimmen, indem mindestens eine aufgrund des mindestens einen Eichwerts festgelegte Konstante gemäß einer Vorschrift mit den beim Messen ermittelten Werten der erstgenannten Größe und der Pulswellengeschwindigkeit verknüpft wird.

13. Verfahren zur Ermittlung des Blutdruckes an einem lebenden Körper, insbesondere unter Verwendung einer Einrichtung nach einem der Ansprüche 1 bis 12, wobei Sensormittel am Körper angeordnet werden, und weiters bei mindestens einem ein arterielles Blutgefäß enthaltenden Meßbereich des Körpers, zumindest eine sich zeitlich im Takt des Pulses ändernde erste Größe ermittelt wird, die ein Maß für die Strömungsgeschwindigkeit, die Durchflußmenge, das Volumen des arteriellen Blutes oder der Durchlaßquerschnittsfläche mindestens eines arteriellen Blutgefäßes und/oder deren Änderung gibt, wobei eine zweite Größe ermittelt wird, die ein Maß für die Pulswellengeschwindigkeit und/oder deren Änderung gibt und durch Verknüpfen der beiden Größen unter Einbeziehung von Eichwerten mindestens eine Größe gebildet wird, die ein Maß für den Blutdruck und/oder dessen Änderung gibt.

14. Verfahren nach Anspruch 13, **dadurch gekennzeichnet**, daß am Anfang einer Meßzeitdauer eine Eichung durchgeführt wird, bei welcher eine einen Hohlraum begrenzende Manschette (73) vorübergehend am Körper befestigt, aufgebläht und entlüftet wird, daß dabei der Druck im Hohlraum gemessen sowie mindestens ein Eichwert ermittelt wird und daß der Blutdruck danach unter Verwendung des mindestens einen Eichwerts bei entfernter Manschette (73) mindestens quasi-kontinuierlich gemessen wird.

**Claims**

1. A device for measuring blood pressure, including sensor means (13, 113, 213, 313, 413, 613) for attachment to a living body, and electronic circuit means (43, 343) connected with the sensor means (13, 113, 213, 313, 413, 613), wherein the sensor means (13, 113, 213, 313, 413, 613) and the circuit means (43, 343) are configured to determine in a measuring region including at least one arterial blood vessel (3, 4, 5, 103, 203, 303) at least one first variable changing over time with the pulse frequency, which is a measure for the flow velocity, the flow quantity, the volume of the arterial blood, or the flow cross section area of at least one arterial blood vessel (3, 103, 203, 303) and/or for a change thereof, and further to determine a second variable which is a measure for the pulse wave velocity and/or a change thereof, and wherein the circuit means (43, 343) are configured to form at least one quantity providing a measure for the blood pressure and/or a change thereof, i.e., by combining the two variables together with calibration values.

2. A device according to claim 1, wherein the sensor means (13, 113, 213, 313, 413, 613) include at least one sensor (21, 23, 121, 123, 221, 223) in order to send sound or lightwaves into the measuring region of the body including at least one arterial blood vessel (3, 4, 5, 103, 203), and to receive sound or lightwaves scattered by the arterial blood, and wherein the circuit means (43) are configured to determine from the scattered sound or lightwaves received by at least one sensor (21, 23, 121, 123, 221, 223) as the first-mentioned variable the flow velocity and/or the flow quantity of the blood flowing through the measuring region.

3. A device according to claim 2, wherein the sensor means (13) are configured to send lightwaves into the at least one measuring region of the body and to receive backscattered lightwaves and to convert them into at least one electrical signal.

4. A device according to claim 3, wherein at least one light source (27) is provided to generate lightwaves whose wavelength is at least 700 nm, at most 1,200 nm and, for example, 800 nm to 1,000 nm.

5. A device according to claim 2, wherein the sensor means (113, 213) include at least one ultrasound transducer (125, 225) in order to send ultrasonic waves into the at least one measuring region of the body and to receive backscattered ultrasonic waves and convert them into at least one electrical signal.

6. A device according to claim 1 or 2, wherein the sensor means (313, 413) include electrodes (329, 429), and the circuit means (343) are configured to determine the electrical impedance or admittance or the change in impedance or admittance over time in the at least one measuring region of the body, and to form therefrom, as the first-mentioned variable, a quantity which is a measure for the volume and/or the flow quantity of arterial blood present in the measuring region and/or for the flow cross section area and/or the inner diameter of at least one blood vessel present in the measuring region.

7. A device according to claim 1 or 2, wherein the sensor means (113) include at least one ultrasound transducer (125) to send ultrasonic waves into the at least one measuring region of the body and to receive reflected ultrasonic waves from at least one wall section of an artery (103), and wherein the circuit means are configured to determine, as the first-mentioned variable a quantity which is a measure for the diameter and/or the flow cross section area and/or for the change in diameter and/or flow cross section area of the artery (103).

8. A device according to any of claims 1 to 7, wherein the sensor means (13, 113, 213, 313, 413) include two sensors (21, 23, 121, 123, 221, 223, 321, 323, 421, 423) for determining, in two measuring regions which are spaced from one another along an artery (3, 103, 203, 303), the changes over time which occur periodically with the pulse frequency in the quantity constituting the first-mentioned variable, and wherein the circuit means (43, 343) are configured to determine from the shift in time between the periodic time changes in said variable as obtained at the two sensors (21, 13, 121, 123), the quantity constituting a measure for the pulse wave velocity.

9. A device according to any of claims 1 to 7, wherein the circuit means are configured to identify a primary extreme (501a) occurring during a pulse period and corresponding to the systolic blood pressure, as well as an additional extreme (501c) caused by the interference between a pulse wave moving away from the heart and a reflected pulse wave, and to determine the time or phase shift between the two extremes (501a, 501c) and to obtain therefrom a quantity constituting a measure for the pulse wave velocity.

10. A device according to any of claims 1 to 7, wherein the sensor means (613) and the circuit means are configured to determine, in addition to the periodically changing quantity determined as the first-mentioned variable in the at least one measuring region of the body, an electrical current of the cardiac muscle, and to form a quantity supplying a measure for the pulse wave velocity and/or a change thereof, from the shift in time between a given feature occurring during each pulse period in the curve of the electrical current of the cardiac muscle over time, and a given feature occurring during each pulse period in the time curve of the quantity determined as the first-mentioned variable, the given features being, for example, the R peak of the characteristic constituting the time curve of the current, or the extreme corresponding to the systolic pressure in the quantity determined as the first-mentioned variable.

11. A device according to any of claims 1 to 10, wherein the circuit means (43, 343) are configured to combine during each measurement, a measured value of the first-mentioned variable and a measured value of the quantity constituting a measure for the pulse wave velocity, to obtain a value for the blood pressure according to a given rule, for example, by assigning a stored function value to at least one of the two measured values and/or, for example, by computing a function value from at least one of the measured values.

12. A device according to any of claims 1 to 11, wherein at least one calibration value input unit (63) is provided and wherein the circuit means (43, 343) are configured to determine the blood pressure, after the input of at least one calibration value as determined by a measurement at the body with a calibration measuring unit (71), at least quasi-continuously, for example for every pulse beat, i.e., by combining according to a rule at least one constant determined on the basis of the at least one calibration value, with the values of the first-mentioned variable obtained during measuring and the pulse wave velocity.

13. A method of determining the blood pressure of a living body, in particular by employing a device according to any of claims 1 to 12, wherein sensor means are arranged on the body, and wherein, in at least one measuring region of the body including an arterial blood vessel, at least one first variable changing over time with the pulse frequency is determined, which is a measure for the flow velocity, the flow quantity, the volume of the arterial blood, or the flow cross section area of at least one arterial blood vessel and/or for a change thereof, and wherein a second variable is determined which is a measure for the pulse wave velocity and/or a change thereof, and wherein at least one quantity is obtained by combining the two variables together with calibration values, thus providing a measure for the blood pressure and/or a change thereof.

14. A method according to claim 13, wherein at the beginning of a measuring period a calibration is made in the course of which a cuff (73) defining a hollow space is temporarily fastened to the body, and subsequently inflated and deflated, during which process the pressure in the hollow space is measured and at least one calibration value is determined, following which the cuff (73) is removed and the blood pressure is measured, at least quasi-continuously, by employing the at least one calibration value.

**Revendications**

1. Système de mesure de la pression du sang, avec des moyens de détection (13, 113, 213, 313, 413, 613) à mettre sur un corps vivant et avec des moyens électroniques de branchement (43, 343), qui sont reliés aux moyens de détection (13, 113, 213, 313, 413, 613), les moyens de détection (13, 113, 213, 313, 413, 613) et les moyens de branchement (43, 343) étant constitués de façon à détecter dans une zone de mesure contenant au moins une poche de sang artérielle (3, 4, 5, 103, 203, 303) au moins une première grandeur, qui varie dans le temps en synchronisme avec les pulsations et qui donne une mesure de la vitesse d'écoulement du sang, de son débit, du volume du sang artériel ou de la surface de la section transversale de passage d'au moins une poche de sang artériel (3, 103, 203, 303) et/ou de sa variation, et de façon à déterminer une seconde grandeur, qui donne une mesure de la vitesse des ondes de pulsation et/ou de leur variation, les moyens de branchement (43, 343) étant en outre constitués pour former, en combinant les deux grandeurs et en incorporant des valeurs d'étalonnage, au moins une grandeur qui donne une mesure de la pression du sang et/ou de sa variation.

2. Système selon la revendication 1, caractérisé en ce que les moyens de détection (13, 113, 213, 313, 413, 613) présentent au moins un détecteur (21, 23, 121, 123, 221, 223), pour rayonner des ondes sonores ou lumineuses dans l'une des zones de mesure du corps contenant au moins une poche de sang artérielle (3, 4, 5, 103, 203) et pour recevoir des ondes sonores ou lumineuses dispersées par le sang artériel et en ce que les moyens de branchement (43) sont constitués pour déterminer, à partir des ondes sonores ou lumineuses dispersées, reçues par au moins un détecteur (21, 23, 121, 123, 221, 223) en tant que première grandeur, la vitesse d'écoulement du sang et/ou le débit du sang qui s'écoule à travers la zone de mesure.

3. Système selon la revendication 2, caractérisé en ce que les moyens de détection (13) sont constitués pour rayonner des ondes lumineuses dans au moins l'une des zones de mesure du corps et pour recevoir les ondes lumineuses dispersées en retour ainsi que pour les convertir en au moins un signal électrique.

4. Système selon la revendication 3, caractérisé en ce qu'il y a au moins un appareil rayonnant de la lumière (27) pour produire des ondes lumineuses, dont la longueur d'onde atteint au moins 700 nm, au maximum 1200 nm, et par exemple 800 nm à 1000 nm.

5. Système selon la revendication 2, caractérisé en ce que les moyens de détection (113, 213) présentent au moins un convertisseur d'ultrasons (125, 225), pour rayonner des ondes d'ultrasons dans au moins l'une des zones de mesure du corps, et recevoir les ondes d'ultrasons dispersées en retour et les convertir en au moins un signal électrique.

6. Système selon la revendication 1 ou 2, caractérisé en ce que les moyens de détection (313, 413) présentent des électrodes (329, 429) et en ce que les moyens de branchement (343) sont constitués pour déterminer l'impédance ou l'admittance électrique ou la variation dans le temps de l'impédance ou de l'admittance d'au moins l'une des zones de mesure du corps, et pour former à partir de là comme première grandeur mentionnée une grandeur qui donne une mesure du volume et/ou du débit du sang artériel, existant dans la zone de mesure et/ou de la surface de la section transversale de passage et/ou du diamètre intérieur d'au moins l'une des poches de sang existant dans la zone de mesure.

**7.** Système selon la revendication 1 ou 2, caractérisé en ce que les moyens de détection (113) présentent au moins un convertisseur d'ultrasons (125), pour rayonner des ondes d'ultrasons dans l'une au moins des zones de mesure du corps et pour recevoir les ondes d'ultrasons réfléchies par au moins une section de paroi d'une artère (103), et en ce que les moyens de branchement sont constitués pour déterminer comme première grandeur mentionnée une grandeur qui donne une mesure du diamètre et/ou de la surface de la section transversale de passage et/ou de la variation du diamètre et/ou de la variation de la surface de la section transversale de passage de l'artère (103).

**8.** Système selon l'une des revendications 1 à 7, caractérisé en ce que les moyens de détection (13, 113, 213, 313, 413) présentent deux détecteurs (21, 23, 121, 123, 221, 223, 321, 323, 421, 423) pour détecter dans deux zones de mesure situées à une certaine distance l'une de l'autre le long d'une artère (3, 103, 203, 303) les variations dans le temps, qui ont lieu périodiquement en synchronisme avec les pulsations des variables qui forment la première grandeur mentionnée et en ce que les moyens de branchement (43, 343) sont constitués pour déterminer à partir du décalage dans le temps entre les variations dans le temps, périodiques, détectées par les deux détecteurs (21, 13, 121, 123) desdites variables, la grandeur qui donne une mesure de la vitesse des ondes de pulsation.

**9.** Système selon l'une des revendications 1 à 7, caractérisé en ce que les moyens de branchement sont constitués pour identifier un point extrême principal (501a) correspondant à la pression systolique du sang, qui se produit pendant une période de pulsation ainsi qu'un point supplémentaire extrême causé par l'interférence d'une onde de pulsation s'éloignant du coeur avec une onde de pulsation réfléchie et pour déterminer le décalage dans le temps entre les deux points extrêmes (501a, 501c) ainsi qu'à partir de là pour former la grandeur qui donne une mesure de la vitesse des ondes de pulsation.

**10.** Système selon l'une des revendications 1 à 7, caractérisé en ce que les moyens de détection (613) et les moyens de branchement sont constitués pour, en addition aux variables qui varient périodiquement, et qui sont détectées comme première grandeur mentionnée dans l'une au moins des zones de mesure du corps, détecter encore un courant électrique du muscle cardiaque et à partir du décalage dans le temps entre un point prédéfini se produisant lors de chaque période de pulsation dans le déroulement dans le temps, qui varie, du courant électrique du muscle cardiaque et un point prédéfini, qui se produit lors de chaque période de pulsation dans le déroulement dans le temps des variables détectées en tant que première grandeur mentionnée, déterminer la grandeur qui donne une mesure de la vitesse des ondes de pulsation et/ou sa variation, les points prédéfinis étant constitués par exemple par la pointe R de la courbe qui représente l'évolution dans le temps du courant ou le point extrême associé à la pression systolique des variables détectées en tant que première grandeur mentionnée.

**11.** Système selon l'une des revendications 1 à 10, caractérisé en ce que les moyens de branchement (43, 343) sont constitués pour, lors de la mesure, exploiter respectivement une valeur mesurée de la grandeur mentionnée en premier et une valeur mesurée de la grandeur qui donne une mesure de la vitesse des ondes de pulsation pour déterminer une valeur de la pression sanguine selon une règle prédéfinie, par exemple en associant une valeur mise en mémoire d'une fonction à au moins l'une des deux valeurs mesurées et/ou par exemple en calculant une valeur de fonction à partir d'au moins l'une des valeurs mesurées.

**12.** Système selon l'une des revendications 1 à 11, caractérisé en ce que l'on a au moins un dispositif d'introduction de valeurs d'étalonnage (63) et en ce que les moyens de branchement (43, 343) sont constitués pour, après l'introduction d'au moins une valeur d'étalonnage, qui a été déterminée par une mesure réalisée sur le corps avec un dispositif de mesure d'étalonnage (71), déterminer la pression du sang de façon au moins quasiment continue, par exemple lors de chaque battement du coeur, en combinant au moins une constante déterminée à partir d'au moins une valeur d'étalonnage, selon une règle, avec les valeurs détectées lors de la mesure de la grandeur mentionnée en premier et de la vitesse des ondes de pulsation.

**13.** Procédé de détermination de la pression du sang sur un corps vivant, en particulier en utilisant un système selon l'une des revendications 1 à 12, dans lequel des moyens de détection sont disposés sur le corps et en outre on détecte dans au moins une zone de mesure du corps, contenant une poche de sang artériel, une première grandeur qui varie dans le temps en synchronisme avec les pulsations, première grandeur qui donne une mesure de la vitesse d'écoulement du sang, de son débit, du volume du sang artériel ou de la surface de la section transversale de passage d'au moins une poche de sang artériel et/ou de sa variation, une seconde grandeur étant déterminée, seconde grandeur qui donne une mesure de la vitesse des ondes de pulsation et/ou de leur variation, et par combinaison des deux grandeurs précédentes, en incorporant des valeurs détalonnage, au moins une grandeur étant formée, qui donne une mesure de la pression du sang et/ou de sa variation.

14. Procédé selon la revendication 13, caractérisé en ce que l'on effectue au début d'une période de mesure un étalonnage, dans le cas duquel on fixe transitoirement sur le corps une manchette (73) qui délimite une poche on gonfle et on dégonfle la manchette, en ce que l'on détermine ce faisant la pression mesurée dans la poche ainsi qu'au moins une valeur d'étalonnage et en ce que l'on mesure ensuite de façon au moins quasi continue la pression du sang en utilisant l'une au moins des valeurs détalonnage après avoir enlevé la manchette (73).

Fig.1

Fig.2

Fig.3

Fig.4

Fig.5

Fig.6

Fig.7

Fig.8

Fig.9

Fig.10